# EUROPEAN PATENT APPLICATION

(11) **EP 0 638 644 A1**
(43) Date of publication of application: **15.02.1995**
(21) Application number: 94110657.7
(22) Date of filing: 08.07.1994
(51) Int. Cl.: C12N 15/12, C07K 14/715, C07K 16/28, A61K 38/17, A61K 39/395, G01N 33/68

(54) **Receptors of interleukin-12 and antibodies**

(30) Priority: 19.07.1993 US 94649; 19.07.1993 US 94713; 31.05.1994 US 248532
(71) Applicant: F. HOFFMANN-LA ROCHE AG, CH-4002 Basel (CH)
(72) Inventor: Chizzonite, Richard Anthony, South Kent Connecticut 06785 (US); Chua, Anne On, Wayne New Jersey 07470 (US); Gubler, Ulrich Andreas, Glen Ridge New Jersey 07028 (US); Truitt, Theresa Patricia, Bloomfield New Jersey 07003 (US)
(74) Representative: Mezger, Wolfgang, Dr.

(57) **Abstract**

This invention relates to Interleukin-12 receptor cDNAs, proteins and uses thereof.

## Description

This invention relates generally to cytokine receptors and more specifically relates to Interleukin-12 receptors.

In order for a molecule such as IL-12 to exert its effect on cells, it is now accepted by those skilled in the art that the molecule must interact with molecules, located on cell membranes, referred to as receptors. Patents which exemplify disclosures of interleukin receptors include Honjo et al., U.S. Patent No. 4,816,565; Urdal et al., U.S. Patent No. 4,578,335; Dower et al., U.S. Patent No. 5,180,812; and Taniguchi et al., U.S. Patent No. 5,198,359.

Also soluble forms of interleukin receptors are known (Fanslow, W.C. et al. (1990) Science 248, 739-41). The effect of Interleukin-1 (IL-1) *in vivo* could be regulated via the administration of a soluble form of its receptor. The results that Fanslow report demonstrate the ability of a soluble cytokine receptor (soluble IL-1R) to modulate biological activity upon exogeneous administration *in vivo*, presumably by acting as a neutralizing agent for the endogeneously produced, corresponding ligand (IL-1), and provides evidence of the therapeutic potential of soluble cytokine receptors in a variety of clinical disorders. Systemic administration of a soluble, extracellular portion of the receptor for IL-1 (soluble IL-1R) had profound inhibitory effects on the development of *in vivo* alloreactivity. Survival of heterotopic heart allografts was prolonged from 12 days in controls to 17 days in mice treated with soluble IL-1R. Lymph node hyperplasia in response to localized injection of allogeneic cells was completely blocked by soluble IL-1R treatment.

Interleukin-12 (IL-12), formerly known as cytotoxic lymphocyte maturation factor or natural killer cell stimulatory factor, is a 75-KDa heterodimeric cytokine composed of disulfide-bonded 40-KDa (p40) and 35-KDa (p35) subunits that has pleiotropic activities including stimulation of the proliferation of activated T and NK cells (Gately, M. K., et al. (1991) J. Immunol., 147, 874) (Kobayashi, M. et al. (1989) J. Exp. Med., 170, 827), enhancement of the lytic activity of NK/LAK cells (Kobayashi, M., et al., supra; Stern, A.S., et al. (1990) Proc. Natl. Acad. Sci. USA, 87, 6808), enhancement of cytolytic T-cell responses (M. Gately et al. (1992) Cell. Immunology, 143, 127), induction of interferon gamma by resting and activated T-and NK-cells (M. Kobayashi et al., supra; S. H. Chan et al. (1991) J. Exp. Med., 173, 869), and promotion of Tₕ1-type helper cell responses (R. Manetti et al. (1993) J. Exp. Med., 177, 1199; C.-S. Hsieh et al. (1993) Science 260, 547).

The biological activity of IL-12 is mediated by the binding of the IL-12 molecules to cell surface, or plasma membrane, receptors on activated T- and NK cells; however, the contributions of the individual subunits, p35 and p40, to receptor binding and signal transduction remain unknown. Studies with labelled IL-12 have shown that this binding occurs in a specific and saturable manner. IL-12 delivers a signal to target cells through a receptor that was initially characterized on PHA-activated CD4+ and CD8+ T-cells and on IL-2 activated CD56+ NK-cells (R. Chizzonite et al. (1992) J. Immunol., 148, 3117; B. Desai, et al. (1992) J. Immunol., 148, 3125). A survey of over 20 human cell lines belonging to the T-, B-, NK- and myelomonocytic lineages only identified a single CD4+, IL-2 dependent human T-cell line (Kit 225) that constitutively expresses the IL-12 receptor and responds to IL-12 (B. Desai, et al. (1992) J. Immunol., 148, 3125; B. Desai, et al. (1993) J. Immunol. 150, 207A). Freshly prepared PHA-activated PBMC and the Kit 225 cell line thus represent two convenient cell sources to study the biochemistry of the functional IL-12 receptor; there may be others. Equilibrium binding experiments with ¹²⁵I-labelled IL-12 showed that i) PHA-activated PBMC express several thousand IL-12 receptors which show 3 classes of affinities: high = 5-20 pM, intermediate = 50-200 pM and low = 2-6 nM; ii) IL-12 receptor expression on PBMC is upregulated by mitogen or IL-2 stimulation; and iii) the IL-12 receptor upregulation correlates with the ability of the cells to proliferate in response to IL-12 (R. Chizzonite, et al. (1992) J. Immunol., 148, 3117; B. Desai, et al. (1992) J. Immunol., 148, 3125). It was not clear at this point whether the biologically functional IL-12 receptor consists of one or more subunits. Affinity crosslinking of labelled IL-12 to activated PBMC demonstrated the size of the cell surface IL-12 binding protein(s) under nonreducing conditions to be in the range of about 150 KDa to about 200 KDa. Additional affinity crosslinking and immunoprecipitation experiments with unlevelled IL-12 bound to ¹²⁵I-surface labelled activated PBMC identified an IL-12 binding protein that under reducing conditions had a size of about 110 KDa (R. Chizzonite, et al. (1992) I. Immunol., 148, 3117).

The present invention is directed towards an isolated cDNA encoding a human low affinity IL-12 receptor protein or subunit thereof. When expressed in mammalian cells, the cDNA gives rise to substantially homogeneous IL-12 receptor protein that binds IL-12 in a specific and saturable manner with an apparent affinity of about 2-10 nM, preferably 2-5 nM. Further, this invention relates to novel antibodies against the interleukin-12 receptor. Representative anti-interleukin-12 antisera provided in accordance with the present invention block interleukin-12 binding to cells expressing interleukin-12 receptors, and can also neutralize interleukin-12 activity.

### Brief description of the drawings:

Figure 1: DNA sequence of human IL-12 receptor cDNA clone No. 5. (translated portion = nucleotides 65 to 2050) (SEQ ID NO:1).
Figure 2: Amino acid sequence of human IL-12 receptor protein as deduced from cDNA sequence of Figure 1. (underlined amino acid residues of N-terminal sequence = signal peptide sequence; amino acid residues nos. 541 to 571 = transmembrane area marked by ------; 6 potential N-linked glycosylation sites in the extracellular portion marked by -------; conserved areas 1 and 2 of the cytoplasmic domain are marked by 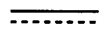 [amino acid residues nos. 577 to 584 and 618 to 629] (SEQ ID NO:2).

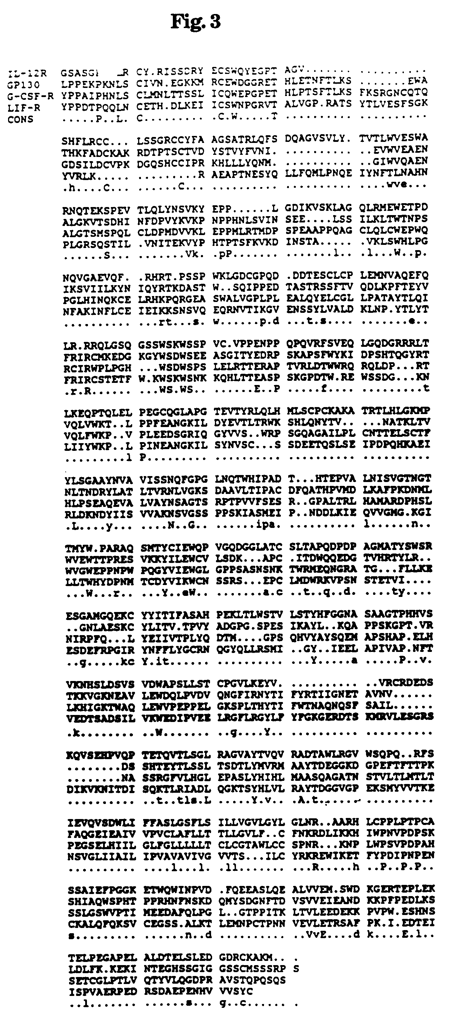

Figure 3: Alignment of the IL-12 receptor protein subunit sequence with human gp130, human granulocyte colony-stimulating factor-receptor (G-CSF-R) and leukemia inhibitory factor-receptor (LIF-R), and resulting consensus sequence. Consensus residues indicated by lowercase letters refer to identities between IL-12 receptor and gp130 only. The following sequence ranges were used: IL-12 receptor protein (SEQ ID NO:2): residues 42-662; gp130: residues 124-742 (Hibi et al. (1990) Cell, 63, 1149); G-CSF-R: residues 98-731 (Fukunaga, et al. (1990) Proc. Natl. Acad. Sci. USA, 87, 8702); LIF-R: residues 331-950 (Gearing, et al. (1991) EMBO J., 10, 2839).
Figure 4A: Scatchard analysis of IL-12 binding to recombinant human IL-12 receptor expressed in COS cells.
Figure 4B: Scatchard analysis of 2-4E6 antibody binding to recombinant human IL-12 receptor expressed in COS cells.
Figure 5: Analysis of the size of the IL-12 receptor protein under reducing conditions. COS cells were transfected with the IL-12 receptor cDNA and labelled with ³⁵S cysteine as described. Aliquots of cell lysates were immunoprecipitated with different antibodies as follows: Lane 1: negative control Ig; lane 2: 2-4E6 antibody; lane 3: negative isotype matched control antibody 4D6. Marker sizes in KDa are indicated on the left of the figure.

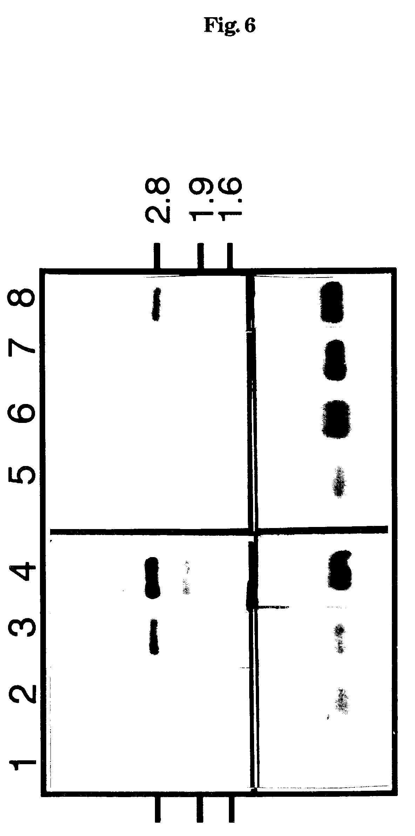

Figure 6: Analysis of the sizes of IL-12 receptor transcripts by RNA blotting. 4 µg of poly A+ RNA were run per lane. Lanes 1-4 and 5-8 represent identical loadings. Lanes 1-4 were probed with a full length cDNA to the receptor, lanes 5-8 with a probe corresponding only to the cytoplasmic portion of the receptor. Lanes 1, 5: unstimulated peripheral blood mononuclear cells (PBMC). Lanes 2, 3, 6, 7: PHA stimulated PBMC. Lanes 1 and 6 represent a 3 day induction of the sample shown in lanes 1 and 5. Samples in lanes 3 and 7 are from an independent 4 day PHA induction and lanes 4 and 8 represent RNA from K6 cells. The bottom panel shows rehybridization of the blots with a probe with a probe for ribosomal protein L32 (loading control).
Figure 7: Inhibition of ¹²⁵I-IL-12 binding to IL-12 (IL-12R) receptor by mouse anti-IL-12R antiserum
Figure 8: Characterization of the IL-12 binding proteins on IL-12R positive human cells by affinity-crosslinking
Figure 9: Immunoprecipitation of the solubilized ¹²⁵I-IL-12/IL-12R crosslinked complex by anti-IL-12R antibodies
Figure 10: Equilibrium binding of ¹²⁵I-2-4E6 to PHA-activated PBMC at room temperature
Figure 11: Equilibrium binding of ¹²⁵I-2-4E6 to human K6 cells at room temperature
Figure 12: Inhibition of ¹²⁵I-2-4E binding to K6 cells by purified 2-4E6 (24E6), human IL-12 (HUIL-12) and control antibody (control IgG)
Figure 13: Equilibrium binding of ¹²⁵I-IL-12 to human K6 cells at room temperature
Figure 14: Equilibrium binding of ¹²⁵I-IL-12 to detergent solubilized IL-12R from K6 cells
Figure 15: Western blot analysis of detergent solubilized IL-12R with mAb 2-4E6
Figure 16: Detection of IL-12 receptor on human cells by flow cytometry
Figure 17: Size of the IL-12 receptor subunit on the surface of transfected COS and CTLL cells. 8% gels were used and marker sizes in KDa are indicated. Lanes 1-4: Analysis of affinity crosslinked complexes under non-reducing conditions. Arrowhead = labelled, uncrosslinked 2-4E6 antibody. Arrows = labelled, uncrosslinked IL-12. Lanes 5-12: Analysis of ¹²⁵I-COS Cell Surface Proteins. Sample reduction, binding of 25 nM unlabelled IL-12 to the cells prior to analysis and use of 1 mM DTSSP crosslinker are indicated below the lanes. Lanes 13-16: Analysis of ¹²⁵I-CTLL cell surface proteins.
Figure 18: Specific inhibition of IL-12-induced lymphoblast proliferation by a rat anti-IL-12R antiserum. (A) Titration by flow cytometry of anti-COS cell antibodies in an anti-IL-12R antiserum (--·--) made against 2-4E6-transfected COS cells, preimmune serum (--o--) from the rat used to prepare the anti-IL-12R antiserum, and a rat antiserum made against COS cells transfected with the human type II IL-1R (-- ■ --). (B-D) Effects of rat sera on proliferation of PHA-activated PBMC induced by IL-12 (B), IL-2 (C), or IL-7 (D). All standard errors were <10% of the means.
Figure 19: Equilibrium binding of ¹²⁵I-IL-12 to COS cells expressing the IL-12 receptor subunit. A) human IL-12 and B) murine IL-12. The insets show analysis of the binding data according to the method of Scatchard.

The present invention is directed to the low affinity receptor of Interleukin-12 (IL-12). The invention includes the homogenous natural IL-12 receptor including allelic variants and subunits as well as functional derivatives thereof: proteins containing a fragment of the natural IL-12 receptor with binding activity for IL-12. Furthermore the present invention includes recombinant IL- 12 receptor proteins as well as fusion proteins, i.e. IL-12 receptor derivatives comprising the amino acid sequence of the natural IL-12 receptor or a partial sequence thereof together with amino acid sequences derived from other proteins. The proteins or polypeptides of the present invention have binding activity for IL-12 as measured by standard assays as described below in the Examples.

A preferred aspect of the present invention relates to a human low affinity IL-12 receptor protein or subunit thereof, more preferred the human receptor protein with the amino acid sequence SEQ ID NO:2 or SEQ ID NO:3.

The invention also includes a soluble form of the IL-12 receptor, preferably a protein exhibiting having IL-12 binding activity, said protein having the amino acid sequence of SEQ ID NO:2 or SEQ ID NO:3 or a portion thereof which encodes a truncated IL-12 receptor protein. Soluble forms of the IL-12 receptors can be prepared by methods known in the art: by isolating soluble IL-12 receptor proteins from culture medium, of IL-12 receptor expressing cells chemical or enzymatic cleavage of IL-12 receptor protein or by recombinant DNA technology.

The availability of the purified receptor, preferably in soluble form, presents therapeutic possibilities as well. Addition of soluble IL-12 receptor interferes with the effect of the interleukin on the cells, since the molecule cannot bind to the cell membrane as freely. Hence, an aspect of the invention is the treatment of pathological conditions caused by excess activity of cells possessing IL-12 receptors by adding an amount of soluble IL-12 receptors sufficient to inhibit binding of IL-12 to the aforementioned cells. This methodology can also be modified, and the soluble receptor can be used as a screening agent for pharmaceuticals. Briefly, a pharmaceutical which works as an IL-12 antagonist can do so by blocking the binding of IL-12 to the IL-12 receptor. Prior to determining whether a material would be effective *in vivo*, one may use the purified IL-12 receptor in connection with a potential pharmaceutical to determine if there is binding. If not, then the pharmaceutical may no longer be a desirable candidate. If there is in fact binding, further testing may be indicated.

The IL-12 receptor proteins of the present invention also include non-naturally occurring IL-12 receptor analogous proteins or mutants. These kind of functional derivatives are proteins in which one or more of the amino acids of the natural IL-12 receptor or its fragments have been replaced or deleted without loss of the IL-12 receptor binding activity. Such analogues may be produced by known methods of peptide chemistry or by recombinant DNA technology.

The term IL-12 receptor protein comprises also derivatives which may be prepared from the functional groups occurring as side chains on the residues or the N- or C-terminal groups, by means known in the art, and are included in the invention as long as they remain pharmaceutically acceptable, i.e. they do not destroy the activity of the protein and do not confer toxic properties on compositions containing it. These derivatives may include, for example, polyethylene glycol side-chains which may mask antigenic sites and extend the residence of the Interleukin-12 receptor protein in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl- or threonyl residues) formed with acyl moieties.

The present invention also relates to cloned DNA sequences coding for the IL-12 receptor and to polynucleotides encoding a protein as defined above, which polynucleotide contains a sequence corresponding to the cDNA encoding the IL-12 receptor, to recombinant vectors comprising a polynucleotide encoding an IL-12 receptor protein, to microorganisms transformed with said recombinant vectors as well as to processes for the preparation of said proteins, polynucleotides and vectors.

The term "polynucleotide containing a sequence corresponding to the cDNA encoding the IL-12 receptor" refers to a polynucleotide containing a sequence which is homologous to or complementary to a sequence in the sequence encoding the IL-12 receptor. The degree of homology to the cDNA will be at least about 80%, preferably at least about 90%.

The practice of the present invention will employ, unless otherwise indicated conventional techniques of molecular biology, microbiology, recombinant DNA and immunology, which are within the skills of an artisan in the field. Such techniques are explained fully in the literature. See e.g. Sambrook, Fritsch & Maniatis, Molecular Cloning; a laboratory Manual (1989) Cold Spring Harbor Laboratory Press 1989.

The DNA sequences and DNA molecules of the present invention may be expressed using a wide variety of host vector combinations. For example, useful expression vectors may consist of segments of chromosomal, non-chromosomal and synthetic DNA sequences. Examples of such vectors are viral vectors, such as the various known derivatives of SV40, bacterial vectors, such as plasmids from *E. coli*, phage DNAs, such as the numerous derivatives of phage λ. M13 and other filamentous single-stranded DNA phages, as well as vectors useful in yeasts, such as the 2µ plasmid, vectors useful in eukaryotic cells more preferably vectors useful in animal cells, such as those containing SV40, adenovirus and/or retrovirus derived DNA sequences.

An aspect of the present invention is a process for preparing a DNA molecule coding for the low affinity Interleukin-12 receptor as described above, characterized in a) the preparation of an anti-Interleukin-12 receptor antibody, b) screening of expression products of a cDNA library, with said antibody, c) identifying and isolating the Interleukin-12 receptor cDNA and d) if desired, preparing a DNA molecule which codes for a functional derivative. A further aspect of the present invention is a process for preparing a low affinity Interleukin-12 receptor protein as described above, characterized in that a) a cell is transformed with a vector comprising a clones gene coding for a low affinity Interleukin-12 receptor protein, b) expression of said protein, c) recovering of said protein and, if desired converting it into a functional derivative thereof.

As used herein, "DNA sequence" refers to a DNA polymer, in the form of a separate fragment or as a component of a larger DNA construct, which has been derived from DNA isolated at least once in substantially pure form, i.e., free of contaminating endogenous materials and in a quantity or concentration enabling identification, manipulation, and recovery of the sequence and its component nucleotide sequences by standard biochemical methods, for example, using a cloning vector. Such sequences are preferably provided in the form of an open reading frame uninterrupted by internal nontranslated sequences, or introns, which are typically present in eukaryotic genes. However, it will be evident that genomic DNA containing the relevant sequences could also be used. Sequences of non-translated DNA may be present 5' or 3' from the open reading frame, where the same do not interfere with manipulation or expression of the coding regions.

As used herein, "expression vector" refers to a plasmid comprising a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription and translation initiation and termination sequences. Structural elements intended for use in various eukaryotic expression systems preferably include a leader sequence enabling extracellular secretion of translated protein by a host cell. Alternatively, where recombinant protein is expressed without a leader or transport sequence, it may include an N-terminal methionine residue. This residue may optionally be subsequently cleaved from the expressed recombinant protein to provide a final product.

The host cell used for the expression of DNA sequence may be selected from a variety of known hosts. Examples for such hosts are prokaryotic or eukaryotic cells. A large number of such hosts are available from various depositories such as the American Type Culture Collection (ATCC) or the Deutsche Sammlung fur Mikro-organismen (DSM). Examples for prokaryotic cellular hosts are bacterial strains such as *E. coli, B. subtilits* and others. Preferred hosts are mammalian cells such as the SV40 transformed African Green monkey kidney cell line COS.

The IL-12 receptor protein produced by fermentation of the prokaryotic and eukaryotic hosts transformed with the DNA sequences of this invention can then be purified to essential homogeneity by known methods such as, for example, by centrifugation at different velocities, by precipitation with ammonium sulphate, by dialysis (at normal pressure or at reduced pressure), by preparative isoelectric focusing, by preparative gel electrophoresis or by various chromatographic methods such as gel filtration, high performance liquid chromatography (HPLC), ion exchange chromatography, reverse phase chromatography and affinity chromatography (e.g. on Sepharose™ Blue CL-6B or on carrier-bound monoclonal antibodies).

The size of the IL-12 receptor subunit at the cell surface was estimated by affinity crosslinking of labelled IL-12 as well as cell-surface labelling studies. Transfected COS cells express the IL-12 receptor subunit as a protein of about 100 KDa size. The calculated molecular weight for the mature form of the protein is 70,426; thus, about 25% of the molecular weight of the surface expressed protein is likely to be carbohydrate. Transfected COS cells also express a larger molecular weight form of the IL-12 receptor subunit.

The available evidence supports the conclusion that the IL-12 receptor dimerization/oligomerization is independent of IL-12 binding. Similar to these findings, it has been reported for the EPO receptor that disulfide-bonded receptor dimers and oligomers are formed and that EPO stimulation had no detectable effect on receptor dimerization (O. Miura, et al. (1993) Archives Biochem. Biophys., 306, 200). Our data also indicate that only the IL-12 receptor dimers/oligomers bind IL-12 with the 2-5 nM affinity observed on intact transfected COS cells. i) An anti-IL-12 antibody only immunoprecipitates an affinity crosslinked complex corresponding to one IL-12 and a receptor dimer/oligomer. ii) Affinity crosslinked complexes of the size expected for one receptor subunit and one IL-12 are formed very inefficiently at IL-12 concentrations corresponding to the K_{D} measured on transfected COS cells. iii) Murine CTLL cells stably expressing the receptor subunit bind IL-12 very inefficiently (estimated K_{D} = 50 nM or lower); these cells also do not express subunit dimers/oligomers. It was unexpected to find that COS cells and CTLL cells differ in their ability to express the IL-12 receptor subunit in a way that allows IL-12 binding. This could be due to species specificity: murine CTLL cells are somehow unable to "process" the human IL-12 receptor protein correctly, resulting in inefficient dimerization/oligomerization and IL-12 binding. Conceivably, COS cells could express an endogenous protein that allows the IL-12 receptor dimerization/oligomerization to occur. Since under the experimental conditions used, the number of low-affinity IL-12 receptor sites per transfected COS cell is always greater than 10⁵, it seems unlikely that an endogenous COS cell component forms dimers or oligomers with the receptor subunit.

The IL-12 receptor subunit is a member of the hemopoietin receptor superfamily. Within that family, it is most closely related over its entire length to gp130 and the receptors for G-CSF and LIF. The extracellular portion of the IL-12 receptor subunit can also be divided into five fibronectin type III repeats, similar to what was reported for gp130 (M. Hibi, et al. (1990) Cell, 63, 1149). It is interesting to note that the ligands for IL-12 receptor and gp130, i.e. IL-12 p40 and IL-6 receptor, both also contain such fibronectin type III repeats (Hibi, et al., supra; Schoenhaut, et al. (1992) J. Immunol., 148, 3433). Some features of the cytoplasmic portion of the IL-12 receptor subunit merit further comment. Compared to the corresponding areas in gp130 (276 AA) and the receptor for LIF (237 AA), it is rather short. However, mutagenesis studies have shown that for gp130, only about 100 amino acids in the cytoplasmic region are sufficient to transduce a signal (Murakami, et al. (1991) Proc. Natl. Acad. Sci. USA, 88, 11349). The potential functionality of the IL-12 receptor cytoplasmic portion appears to be borne out further by the presence of a number of features conserved in other functional hemopoietic receptors (among them the receptors for G-CSF, EPO and GM-CSF): conserved areas 1 and 2 (Murakami, et al. (1991) Proc. Natl. Acad. Sci. USA, 88, 11349) are clearly present and thus give the low affinity IL-12 receptor component the makeup of a beta type subunit (N. Stahl, et al., 1993, Cell, 74:587).

The IL-12 receptor cDNA is useful for the following purposes:
Expression of recombinant IL-12 receptor protein in high levels and its use as an antigen allows production of additional neutralizing monoclonal and polyclonal antibodies. Such neutralizing antibodies can be used in *in vivo* model settings to elucidate the role that IL-12 and its receptor play in normal as well as pathologic immune responses (i.e. disease states that are aggravated by activated T- and NK-cells like autoimmune diseases, graft versus host disease and rheumatoid arthritis).

IL-12 receptor proteins can be administered, for example, for the purpose of suppressing immune responses in a human. A variety of diseases or conditions are caused by an immune response to alloantigen, including allograft rejection and graft-versus-host reaction. In alloantigen-induced immune responses, IL-12 receptor can suppress lymphoproliferation and inflammation which result upon activation of T cells. IL-12 receptor can therefore be used to effectively suppress alloantigen-induced immune responses in the clinical treatment of, for example, rejection of allografts (such as skin, kidney, and heart transplants), and graft-versus-host reactions in patients who have received bone marrow transplants.

IL-12 receptor can also be used in clinical treatment of autoimmune dysfunctions, such a rheumatoid arthritis, diabetes and multiple sclerosis, which are dependent upon the activation of T cells against antigens not recognized as being indigenous to the host. IL-12 receptor can also be useful in treatment of septic shock in which interferon gamma produced in response to IL-12 plays a central role in causing morbidity and mortality (Doherty et al. (1992) J. Immunol. 149, 1666).

Purified IL-12 receptor compositions will be useful in diagnostic assays for IL-12 or IL-12 receptor, and also in raising antibodies to IL-12 receptor for use in diagnosis or therapy. In addition, purified IL-12 receptor compositions may be used directly in therapy to bind or scavenge IL-12, thereby providing a means for regulating the immune or inflammatory activities of IL-12. In its use to prevent or reverse pathologic immune responses, soluble IL-12 receptor can be combined with other cytokine receptor antagonists such as antibodies to the IL-2 receptor, soluble TNF receptor, the IL-1 receptor antagonist, and the like.

The dose ranges for the administration of the IL-12 receptor proteins and fragments thereof may be determined by those of ordinary skill in the art without undue experimentation. In general, appropriate dosages are those which are large enough to produce the desired effect, for example, blocking the binding of endogenous IL-12 to its natural receptor. The dosage should not be so large as to cause adverse side effects, such as unwanted cross-reactions, anaphylactic reactions, and the like. Generally, the dosage will vary with the age, condition, sex and extent of disease in the patient, counter indications, if any, immune tolerance and other such variables, to be adjusted by the individual physician. The expected dose range is about 1 ng/kg/day to about 10 mg/kg/day. The IL-12 receptor proteins and fragments thereof can be administered parenterally by injection or by gradual perfusion over time. They can be administered intravenously, intraperitoneally, intramuscularly, or subcutaneously.

Preparations for parenteral administration include sterile or aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, anti-microbials, anti-oxidants, chelating agents, inert gases and the like. See, generally, *Remington's Pharmaceutical Science,* 18th Ed., Mack Eds., 1990.

The present invention also relates to novel immunoglobulins, which include novel antisera and monoclonal antibodies to the human IL-12 receptor for all three classes of affinities mentioned above.

Representative anti-IL-12R antisera provided in accordance with the present invention block IL-12 binding to cells expressing IL-12R and can also neutralize IL-12 activity. In further embodiments of the present invention, the monoclonal antibodies which are selective to IL-12R are prepared in accordance with generally known techniques, such as the method of Köhler and Milstein. Suitable monoclonal antibodies to IL-12R can be modified by known methods to provide chimeric, humanized or single chain antibody (SCA) embodiments.

The immunoglobulins according to the present invention comprise antibodies which are capable of inhibiting the binding of IL-12 to the IL-12 receptor and are capable of neutralizing IL-12 bioactivity by binding to the IL-12 receptor. Further the invention refers to immunoglobulins which bind to the IL-12 receptor but which are not capable of inhibiting the binding of IL-12 to the IL-12 receptor and are not capable of neutralizing IL-12 bioactivity by binding to the IL-12 receptor.

The IL-12 receptor antibodies of the present invention can be used to determine IL-12 receptor expression on human cells, such as peripheral blood lymphocytes and bone marrow cells, in normal and pathological conditions. The antisera and monoclonal antibodies of the invention can also be used to block IL-12 binding to its receptor and thus block its biologic activity. Neutralizing antibodies of the present invention can thus be used for therapeutic intervention in a number of disease states that are aggravated by activated T-cells and NK cells, such as autoimmune diseases, graft versus host disease and rheumatoid arthritis. Finally, as has been specifically demonstrated by the specific antibody embodiment of the present invention, such antibody will also be useful for expression cloning strategies to isolate a cDNA coding for the IL-12 receptor.

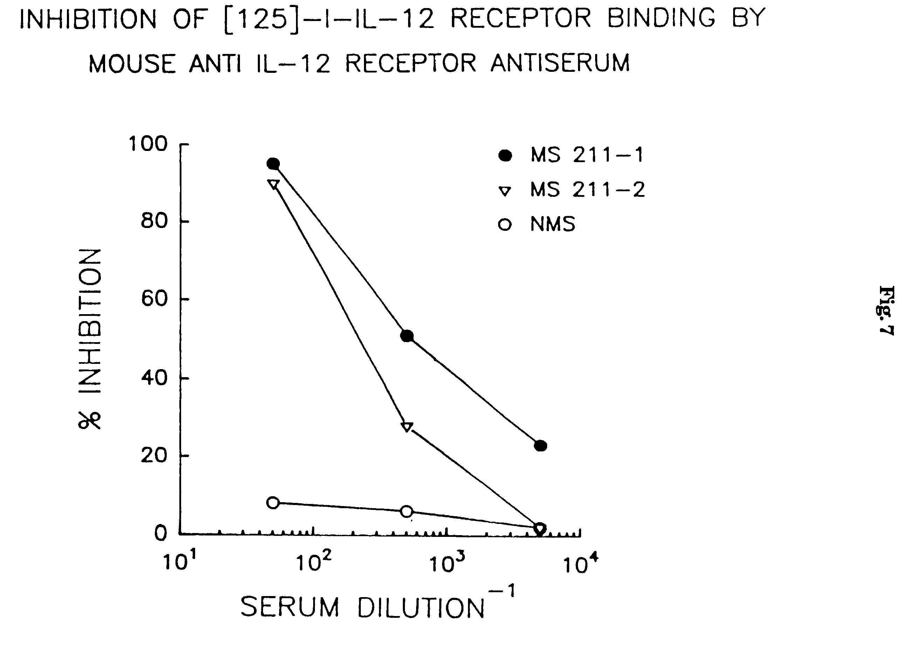

The antisera of the invention can be conveniently produced by immunizing host animals with PHA-activated human PBMC. Suitable host animals include rodents, such as, for example, mice, rats, rabbits, guinea pigs and the like, or higher mammals such as goats, sheep, horses and the like. Initial doses and booster shots can be given according to accepted protocols for eliciting immune responses in animals, e.g., in a preferred embodiment mice received an initial dose of 6 x 10⁷ cells/mouse i.p. and five subsequent booster shots of between 2-5 x 10⁷ cells over a six month period. Immunized mice were observed to develop an immune response against the human IL-12R as determined by inhibition of ¹²⁵I-IL-12 binding to PHA-activated PBMCs (Figure 7) and immunoprecipitation of the complex of ¹²⁵I-IL-12 crosslinked to IL-12R, which methods provide a convenient way to screen for hosts which are producing antisera having the desired activity.

Monoclonal antibodies are produced conveniently by immunizing Balb/c mice according to the above schedule followed by injecting the mice with 1x10⁷ cells i.p. and 2.5 x 10⁶ cells iv. on two successive days starting four days prior to the cell fusion. Other protocols well known in the antibody art may of course be utilized as well. The complete immunization protocol detailed herein provided an optimum protocol for serum antibody response for the antibody to the human IL-12 receptor. Other immunization protocols resulted in a lower serum antibody response than the protocol set forth below: For example, 1) immunization with lower numbers of PHA-activated lymphoblasts (0.7 to 1.8 x 10⁷ cells/booster immunization); 2) immunization with fewer numbers of booster immunization or over a short period of time (40 days) with 2 to 6 x 10⁷ cells/immunization; and 3) immunizations with cell membranes derived from PHA-activated lymphoblasts (membranes equivalent to 1 to 4 x 10⁸ cells/immunization) all produced serum antibody responses but not as significant as the protocol below. Similar results were obtained when immunizing rats.

B lymphocytes obtained from the spleen, peripheral blood, lymph nodes or other tissue of the host may be used as the monoclonal antibody producing cell. Most preferred are B lymphocytes obtained from the spleen. Hybridomas capable of generating the desired monoclonal antibodies of the invention are obtained by fusing such B lymphocytes with an immortal cell line, that is a cell line that which imparts long term tissue culture stability on the hybrid cell. In the preferred embodiment of the invention the immortal cell may be a lymphoblastoid cell or a plasmacytoma cell such as a myeloma cell, itself an antibody producing cell but also malignant. Murine hybridomas which produce IL-12R monoclonal antibodies are formed by the fusion of mouse myeloma cells and spleen cells from mice immunized against hIL-12R expressed on the surface of activated peripheral blood mononuclear cells. Chimeric and humanized monoclonal antibodies can be produced by cloning the antibody expressing genes from the hybridoma cells and employing recombinant DNA methods now well known in the art to either join the subsequence of the mouse variable region to human constant regions or to combine human framework regions with complementarity determining regions (CDR's) from a donor mouse or rat immunoglobulin, EP 0239400). An improved method for carrying out humanization of murine monoclonal antibodies which provides antibodies of enhanced affinities is set forth in International Patent Application No. WO 92/11018.

The invention also includes a process for the preparation of a monoclonal antibody directed to an Interleukin-12 receptor which comprises a) immunizing a host animal with Interleukin-12 receptor, b) fusing B lymphocytes of said host animal with an immortal cell line, and c) preparation of monoclonal antibodies by culturing the resulting hybridoma cell line.

Polypeptide fragments comprising only a portion of the primary antibody structure may be produced, which fragments possess one or more immunoglobulin activities. These polypeptide fragments may be produced by proteolytic cleavage of intact antibodies by methods well known in the art, or by inserting stop codons at the desired locations in expression vectors containing the antibody genes using site-directed mutagenesis to produce Fab fragments or (Fab')₂ fragments. Single chain antibodies may be produced by joining VL and VH regions with a DNA linker ( see Huston et. al., Proc. Natl. Acad. Sci. U.S.A., 85, 5879-5883 (1988) and Bird et. al., Science, 242, 423-426 (1988).

It is also within the skill of the art to utilize the monoclonal antibodies of the present invention as therapeutic agents. They may be formulated for parenteral administration in a manner known in the art such as by dissolving the purified monoclonal antibody product either intact or as a fragment in water for injection and sterile filtering. The dosage form may contain known excipients for parenteral administration of proteins such as buffers, stabilizers and carrier protein. The administered dosage will be selected by the attending physician by giving due consideration to the disease severity and nature as well as the age, size and condition of the patient. As immunoglobulins have demonstrated extended half-lifes in patients dosing every 10-14 days is usually sufficient. It is also within the skill of the art to modify the monoclonal antibody by forming a hybrid with a toxin molecule such as with a pseudomonas exotoxin or with the A chain of ricin to provide a hybrid molecule capable of destroying the cells expressing the IL-12R in a selective manner.

The invention also pertains to a method for detecting peripheral blood cells which express the IL-12 receptor, which comprises contacting a sample which contains the subject cells with substances capable of forming complexes with the IL-12 receptors so as to form cellular complexes between the substances and the IL-12 receptors, and detecting such cellular complexes. Another embodiment of the invention provides a method of evaluating cell activity in a subject which comprises detecting peripheral blood cells according to the method described above.

In the preferred embodiments, the substances are capable of forming complexes only with the IL-12 receptors present on the surface of peripheral blood cells in which the receptors were expressed. Particularly preferred are substances which comprise IL-12 monoclonal antibody.

One embodiment of the invention provides a method of evaluating immune cellular activity which comprises:
a. isolating peripheral blood mononuclear cells;
b. treating the cells with the IL-12 monoclonal antibody; and
c. determining the amount of monoclonal antibody bound to the cells.

The invention also involves a method for diagnosing an immune system abnormality in a subject which comprises determining the number of T cells, NK cells, or B-cells in a sample derived from the subject. This method involves contacting the sample with substances capable of forming complexes with the IL-12 receptors and determining the percentage of T cells, NK cells or B cells in the sample which have the IL-12 receptor. Comparing the percentages so determined with the percentage of cells which have the IL-12 receptor in a sample from a normal subject who does not have the immune system abnormality, the differences in the percentage of cells so determined being indicative of the immune system abnormality. Preferably, the subject is an animal, e.g., a human.

As a molecule associated with T cell, NK cell and B cell function, the measurement of IL-12R expression has diagnostic importance. Because IL-12R is distinctive to activated T cells, NK cells or B cells, it is a unique marker for these cells in a population of lymphocytes.

Moreover, the level of expression of IL-12R provides a measure of T cell, NK cell or B cell activity. This information may be important for evaluating the immune status of an individual. For instance, in the treating of certain disease, such as cancer, agents which affect the immunocompetency are often used. Assays for IL-12R expression may allow physicians to monitor the immune status of the patient and to adjust treatment to minimize the risk of opportunistic infection, often a threat to immunocompromised patients.

Assays for IL-12R expression may be conventional immunochemical assays for cell surface antigens. Peripheral blood mononuclear cells can be isolated from patient and incubated with IL-12R monoclonal antibody under conditions which allow the antibody to bind the surface antigen. Antibody bound to the cell surface provides a measure of IL-12R expression. Binding of the antibody to cells may be evaluated by employing an IL-12R monoclonal antibody labelled with a radioactive, fluorescent or other compound capable of being detected.

The invention also involves a method for detecting soluble IL-12 receptor concentration in samples derived from subjects with immune system disorders, cancer, or other diseases that would be marked by an increase or decrease in soluble form of IL-12R. Assays for soluble IL-12R may be conventional sandwich immunochemical assays or ¹²⁵I-IL-12 binding assays to immobilized IL-12R.

The terms and expressions which have been employed are used as terms of description and not of limitation, and there is no intention in the use of such terms and expressions of excluding any equivalents of the features shown and described or portions thereof, it being recognized that various modifications are possible within the scope of the invention. Certain embodiments of this invention are exemplified in the Examples which follow. The Examples are set forth to aid in an understanding of the invention but are not intended to, and should not be construed to, limit in any way the invention as set forth in the claims which follow.

### MATERIALS AND METHODS

### Proteins and Plasmids

Recombinant human IL-12 (U. Gubler et al., Proc. Natl. Acad. Sci. USA (1991) 88, 4143, and murine IL-12 (D. Schoenhaut et al., J. Immunology (1992), 148, 3433) were obtained as described therein.

The murine anti human IL-12 receptor monoclonal antibody 2-4E6 used herein was generated as described herein below and was purified from ascites fluids by affinity chromatography on protein G-agarose according to the manufacturer's instructions (Genex). The proteins were labelled with I-125 by a modification of the Iodogen method as described (Pierce Chemical Co., Rockford, IL). Radiospecific activities of 5000 - 7000 cpm/fmole for IL-12 and 1500 - 2500 cpm/fmole for the 2-4E6 antibody were typically obtained.

The plasmid pEF-BOS is based on a pUC 119 backbone and contains the elongation factor 1 alpha promoter to drive expression of genes inserted at the BstXI site (S. Mizushima and S. Nagata (1990) Nucl. Acids Res. 18, 5322).

### Example 1

### Preparation. Characterization and Purification of Hybridoma Antibodies

Balb/c mice (Charles River Laboratories) were immunized by the intraperitoneal route with PHA(phytohemagglutanin)-activated human PBMC (PHA-activated PBMC) at 6 x 10⁷ cells/mouse. Mice received 5 subsequent booster injections of between 2-5 x 10⁷ cells over a six month period. For preparation of activated spleen cells, 2 mice were injected intraperitoneally and intravenously with 1 x 10⁷ and 2.5 x 10⁶ cells, respectively, on two successive days, starting four days prior to the cell fusion. Spleen cells were isolated from these mice and fused with SP2/0 (ATCC CRL 1581) cells at a ratio of 1:1 with 35% v/v polyethylene glycol 4000 (E. Merck) according to the method of Fazekas et al., J. Immunol. Methods (1980) 35, 1. The fused cells were plated at a density of 6 x 10⁵ cells/ml/well in 48-well cluster dishes in IMDM supplemented with 10% FBS, glutamine (2 mM), β-mercaptoethanol (0.1 mM), gentamicin (50 g/ml), 5% ORIGEN hybridoma cloning factor (IGEN, Inc.), 5% P388D1 (ATCC CRL 46) supernatant (Nordan, R.P., et al., J. Immunol. (1987) 139, 813) and 100 Units/ml rHuIL-6. Hybridoma supernatants were assayed for specific anti-IL-12 receptor antibodies by: 1) immunoprecipitation of the soluble complex of ¹²⁵I-HulL-12 crosslinked to IL-12 receptor (125I-IL-12/IL-12R), 2) inhibition of ¹²⁵I-HuIL-12 binding to PHA-activated PBMC's, and 3) differential binding to IL-12 receptor positive cells versus receptor negative cells). Hybridoma cell lines secreting specific anti-receptor antibodies were cloned by limiting dilution. Antibodies were purified from ascites fluids by affinity chromatography on Protein G bound to cross-linked agarose according to the manufacturer's protocol (Genex).

### Example 2

### Preparation of Human PHA Lymphoblasts and IL-12 Receptor Binding Assays

Human peripheral blood mononuclear cells were isolated (see Gately et al, J. Natl. Cancer Inst. 69, (1982) 1245) and cultured at 37°C at a density of 5 x 105 cells/ml in tissue culture medium (TCM) containing 0.1% PHA-P (Difco). After 3 days, the cultures were split 1:1 with fresh TCM, and human rIL-2 was added to each culture to give a final concentration of 50 units/ml. The cultures were then incubated for an additional 1-2 days, prior to use in assays.

PHA-activated human PBMC were washed once in binding buffer (RPMI-1640, 5% FBS, 25 mM HEPES pH 7.4) and resuspended in binding buffer to a cell density of 7 x 10⁶ cells/ml. Lymphoblasts (7 x 10⁵ cells) were incubated with various concentrations of ¹²⁵I-IL-12 (5-10000 pM) at room temperature for the designated times. Cell bound radioactivity was separated from free ¹²⁵I-IL-12 by centrifugation of the assay mixture through 0.1 ml of an oil mixture (1:2 mixture of Thomas Silicone Fluid 6428-R15 : A.H. Thomas, and Silicone Oil AR 200 : Gallard-Schlessinger) at 4°C for 90 sec at 10,000 x g. The tip containing the cell pellet was excised, and cell bound radioactivity was determined in a gamma counter. Non-specific binding was determined by inclusion of 100 nM unlabelled IL-12 in the assay. Incubations were carried out in duplicate or triplicate. Receptor binding data were analyzed by using the non-linear regression programs EBDA and LIGAND as adapted for the IBM personal computer by McPherson, J. Pharmacol. Methods (1985) 14, 213 from Elsevier-BIOSOFT.

### Example 3

### Affinity Cross-Linking of ¹²⁵I-IL-12 to IL-12 Receptor Bearing Cell Lines

IL-12 receptor bearing cells were incubated with ¹²⁵I-IL-12 (100-500 pM) in the presence or absence of excess unlabelled IL-12 for 2 hr at room temperature. The cells were then washed with ice-cold PBS pH 8.3 (25 mM Sodium Phosphate pH 8.3, 0.15 M NaCl and 1 mM MgCl₂) and resuspended at a concentration of 0.5-1.0x10⁷ cells/ml in PBS pH 8.3. BS3 (Pierce) in dimethyl sulfoxide was added to a final concentration of 0.4 mM. Incubation was continued for 30 min. at 4°C with constant agitation. The cells were washed with ice-cold 25 mM Tris-HCl (pH 7.5), 0.15 m NaCl and 5 mM EDTA and then solublized at 0.5 - 1.0 x 10⁸ cells/ml in solubilization buffer (50 mM Tris-HCl (pH 8.0) containing 8 mM CHAPS, 0.25 M NaCl, 5 mM EDTA, 40 µg/ml PMSF, 0.05% NaN₃, and 1% BSA) for 1 hr at 4°C. The extracts were centrifuged at 12,000 x g for 45 min. at 4°C to remove nuclei and other debris.

### Example 4

### Immunoprecipitation Assay of the Soluble Complex of ¹²⁵I-IL-12 Crosslinked to Human IL-12R

For the immunoprecipitation assay, hybridoma culture supernatant (0.5 ml), diluted antisera, or purified IgG was added to a microfuge tube containing 0.1 ml of a 50% suspension of either goat-anti-mouse IgG coupled to agarose (SIGMA CHEM. CO.) or Protein G coupled to Sepharose 4B (Pharmacia). The assay volume was brought up to 1.0 ml with IP buffer (8 mM CHAPS in PBS (0.25 M NaCl), 1% BSA, & 5 mM EDTA) and the mixture was incubated on a rotating mixer for 2 hr at room temperature. The beads were pelleted by centrifugation, resuspended in 1 ml IP buffer containing ¹²⁵I-IL-12/IL-12R (10-20,000 cpm) and the mixture was incubated on a rotating mixer for 16 hr at 4°C. After this incubation, the beads were pelleted by centrifugation and washed twice in IP buffer without BSA. The ¹²⁵I-labelled receptor complex bound to the solid phase antibodies was released by adding 100 µl of 2x Laemmli sample buffer (Nature 227, 680 (1970)) with and without 10% β-mercaptoethanol and heating for 5 min. at 95°C. The immunoprecipitated proteins were analyzed by SDS-PAGE on 8% or 4-15% gradient polyacrylamide gels and visualized by autoradiography.

### Example 5

### Assays for IL-12R Solubilized from Cells Expressing IL-12 Receptor.

To confirm that the antibodies identified by the immunoprecipitation assay were specific for IL-12R, an immunoprecipitation/soluble IL-12R binding assay was developed. As described in Example 1 above, antibodies (as hybridoma supernatant, purified IgG (50 µg) or antisera) were immobilized by binding to goat anti-mouse IgG coupled to agarose (100 µl; Sigma Chemical Co.) or protein G coupled to Sepharose 4B (100 µl; Pharmacia). For some experiments, antibodies were covalently crosslinked to protein G-Sepharose 4B, before being used in the assay. See Stem and Podlaski, Techniques in Protein Chemistry (1993). The immobilized antibodies were resuspended in IP buffer (0.3 ml) and 0.2 ml of a detergent solubilized extract of PHA-activated PBMCs or K6 cells that contained IL-12R was added. To prepare the detergent solubilized IL-12R preparation, the cells were washed with ice-cold 25 mM Tris-HCl (pH 7.5), 0.15 M NaCl and 5 mM EDTA and then solublized at 1.5 x 10⁸ cells/ml in solubilization buffer (50 mM Tris-HCl, pH 8.0, containing 8 mM CHAPS, 0.25 M NaCl, 5 mM EDTA, 40 µg/ml PMSF, 0.05% NaN₃, and 1% BSA) for 1 hr at 4°C. The extracts were centrifuged at 120,000 x g for 60 min. at 4°C to remove nuclei and other debris. The mixture was incubated on a rotating mixer for 16 hr at 4°C. After this incubation, the beads were pelleted by centrifugation and resuspended in IP buffer (0.15 ml) containing ¹²⁵I-HuIL-12 at concentrations ranging from 0.05 to 7.5 nM. The IL-12R immobilized on the antibody coated beads was incubated with ¹²⁵I-HulL-12 for 2 hrs. at room temperature on a shaker. Following this incubation, the beads were pelleted, washed twice with IP buffer and the bound radioactivity determined in a gamma counter. Nonspecific binding was determined by inclusion of 70 nM unlabelled human IL-12 in the assay. Solubilized IL-12R binding data were analyzed according to the method of Scatchard, (Assn. N.Y. Acad. Sci. 51, 660 (1949)) by using the nonlinear regression programs EBDA and Ligand as adapted for the IBM PC by McPherson, supra from Elsevier-BIOSOFT.

### Example 6

### Competitive Inhibition of ¹²⁵I-IL-12 Receptor Binding by Antibodies

The ability of hybridoma supernatant solutions, purified IgG, or antisera to inhibit the binding of ¹²⁵I-IL-12 to PHA-activated lymphoblasts was measured as follows: serial dilutions of culture supernatants, purified IgG or antisera were mixed with activated lymphoblasts (1-1.5 x 10⁶ cells) in binding buffer (RPMI-1640, 5% FBS + 25 mM HEPES pH 7.4) and incubated on an orbital shaker for 1 hour at room temperature. ¹²⁵I-HuIL-12 (1 x 10⁵ cpm) was added to each tube and incubated for 1-2 hours at room temperature. Non-specific binding was determined by inclusion of 10 nM unlabelled IL-12 in the assay. Incubations were carried out in duplicate or triplicate. Cell bound radioactivity was separated from free ¹²⁵I-IL-12 by centrifugation of the assay through 0.1 ml of an oil mixture as described above. The tip containing the cell pellet was excised, and cell bound radioactivity was determined in a gamma counter.

### Example 7

### Labelling of Human IL-12 and Mab 2-4E6 with ¹²⁵I

Human IL-12 and purified 2-4E6 (Example 13) IgG were labelled with ¹²⁵I by a modification of the Iodogen method (Pierce Chemical Co., Rockford, IL). Iodogen was dissolved in chloroform and 0.05 mg dried in a 12 x 15 mm borosilicate glass tube. For radiolabeling, 1.0 mCi Na[¹²⁵I] (Amersham, Chicago, IL) was added to an Iodogen-coated tube containing 0.05 ml of Tris-iodination buffer (25 mM Tris-HCl pH 7.5, 0.4 M NaCl and 1 mM EDTA) and incubated for 4 min at room temperature. The activated ¹²⁵I solution was transferred to a tube containing 0.05 to 0.1 ml IL-12 (7 µg) or IgG (100 µg) in Tris-iodination buffer and the reaction was incubated for 9 min. at room temperature. At the end of the incubation, 0.05 ml of Iodogen stop buffer (10 mg/ml tyrosine 10% glycerol in Dulbecco's PBS, pH 7.40) was added and reacted for 3 min. The mixture was then diluted with 1.0 ml Tris-iodination buffer, and applied to a Bio-Gel P10DG desalting column (BioRad Laboratories) for chromatography. The column was eluted with Tris-iodination buffer, and fractions (1 ml) containing the peak amounts of labelled protein were combined and diluted to 1 x 108 cpm/ml with 1% BSA in Tris-iodination buffer. The TCA preciptable radioactivity (10% TCA final concentration) was typically in excess of 95% of the total radioactivity. The radiospecific activity was typically ∼ 1500 to 2500 cpm/fmol for 2-4E6 IgG and 5000 to 7000 cpm/fmol for IL-12.

### Example 8

### Binding Assays of ¹²⁵I-2-4E6 to Intact Cells

PHA-activated human PBMC were washed once in binding buffer (RPMI 1640, 5% FBS and 25 mM HEPES, pH 7.4) and resuspended in binding buffer to a cell density of 1.5 x 10⁷ cells/ml. Lymphoblasts (1.5 x 10⁶ cells) were incubated with various concentrations of ¹²⁵I-2-4E6-IgG (0.005 to 2 nM) at room temperature for 1.5 hrs. Cell bound radioactivity was separated from free ¹²⁵I-2-4E6 IgG by centrifugation of the assay mixture through 0.1 ml silicone oil at 4°C for 90 seconds at 10,000 x g. The tip containing the cell pellet was excised, and cell bound radioactivity was determined in a gamma counter. Non-specific binding was determined by inclusion of 67 nM unlabelled 2-4E6 IgG in the assay. Incubations were carried out in duplicate or triplicate. Receptor binding data were analyzed by using the nonlinear regression programs EBDA, Ligand and Kinetics as adapted for the IBM personal computer by McPherson, supra from Elsevier BIOSOFT.

### Example 9

### Expression of Recombinant IL-12R in COS Cells and Determination of ¹²⁵I-2-4E6 Binding

COS cells (4-5 x 10⁷) were transfected by electroporation with 25 µg of plasmid DNA expressing recombinant human IL-12R, as describe herein below, in a BioRad Gene Pulser (250 µF, 250 volts) according to the manufacturer's protocol. The cells were plated in a 600 cm² culture plate, harvested after 72 hours by scraping, washed and resuspended in binding buffer. Transfected cells (8 x 10⁴ were incubated with increasing concentrations of ¹²⁵I-labelled 2-4E6 or IL-12 at room temperature for 2 hrs. Cell bound radioactivity was separated from free ¹²⁵I-labelled 2-4E6 or IL-12 as described above.

### Example 10

### Western Blot Analysis of Soluble IL-12R with mAb 2-4E6

PHA-activated PBMC were washed 3 times with ice-cold PBS and solubilized at 0.5 - 1 x 10⁸ cells/ml in solubilization buffer (50 mM Tris-HCl pH 8.0 containing 8 mM CHAPS, 0.25 M NaCl, 5 mM EDTA, 40 µg/ml PMSF, 0.05% NaN₃ and 1 mg/ml BSA) for 1 hr at 4°C. The extracts were centrifuged at 12,000 x g for 45 min. at 4°C to remove nuclei and other debris. The extracts were incubated with 2-4E6 IgG or control IgG bound to goat-anti-mouse IgG immobilized on cross-linked agarose (Sigma Chemical Co.). The precipitated proteins were released by treatment with 0.1 M glycine pH 2.3, neutralized with 3M Tris, mixed with 1/5 volume of 5 x Laemmli sample buffer, and separated by SDS/PAGE on 8% pre-cast acrylamide gels (NOVEX). The separated proteins were transferred to nitrocellulose membrane (0.2 µM) for 16 hours at 100 volts in 10 mM TRIS-HCl (pH 8.3), 76.8 mM glycine, 20% methanol and 0.01% SDS. The nitrocellulose membrane was blocked with BLOTTO (5.0% w/v nonfat dry milk in PBS + 0.05% Tween 20) and duplicate blots were probed with ¹²⁵I-2-4E6 IgG (1 x 106 cpm/ml in 8 mM CHAPS in PBS, 0.25 M NaCl, 10% BSA and 5 mM EDTA) + unlabelled 2-4E6 IgG (67 nM).

### Example 11

### Analysis of IL-12 Receptor Expression on Human Cells by Fluorescence Activated Cell Sorting with mAb 2-4E6

To stain cells expressing IL-12 receptor, 1 x 10⁶ cells in 100 µl staining buffer (PBS containing 2% FBS and 0.1% NaN₃) were incubated with 10 µl of 2-4E6 ascites fluid for 25 min. at 4°C. Cells were then washed twice with staining buffer followed by incubation with a 1:100 dilution of goat F(ab)2 anti mouse Ig-PE (Tago, Burlingame CA) for 25 min. at 4°C. The stained cells were washed twice with staining buffer and then analyzed on a FACScan flow cytometer (Becton Dickinson).

### Example 12

### Inhibition of IL-12 Binding to Human PHA-Lymphoblasts by Mouse Anti-IL-12R Antiserum

Mice immunized with PHA-activated PBMCs developed an immune response against the human IL-12R as determined by inhibition of ¹²⁵I-IL-12 binding to PHA-activated PBMCs (Figure 7) and immunoprecipitation of the complex of ¹²⁵I-IL-12 crosslinked to IL-12R (data not shown). The dilutions for half-maximal inhibition of ¹²⁵I-IL-12 binding to PHA-activated PBMCs were 1/500 and 1/250 for animals 211-1 and 211-2, respectively (Figure 7). These antisera also neutralized IL-12 biologic activity as measured in a PHA-lymphoblast proliferation assay (data not shown). Spleen cells isolated from these mice were fused with SP2/0 myeloma cells and the resulting hybridomas were initially screened for IL-12R specific antibodies by immunoprecipitation of the ¹²⁵I-IL-12/IL-12R complex and by inhibition of ¹²⁵I-IL-12 binding to IL-12R.

For Figure 7, ten fold serial dilutions of mouse anti-IL-12R immune serum (#211-1 and #211-2) and normal mouse serum (NMS) were preincubated with PHA-activated PBMC for 60 min at RT (room temperature) before addition of ¹²⁵I-IL-12 (100 pM). After addition of ¹²⁵I-IL-12, the reaction was incubated for 1-2 hrs at RT and the cell bound radioactivity was determined. The data are expressed as the % Inhibition of ¹²⁵I-IL-12 binding in the presence of the immune serum when compared to the specific binding in the absence of serum.

### Example 13

### Identification and Characterization of Monoclonal Anti-IL-12R Antibodies

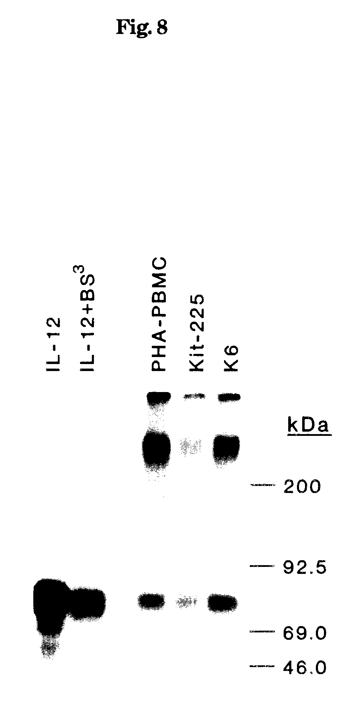

The immunoprecipitation assay identified 13 hybridomas secreting putative non-neutralizing anti-IL-12R antibodies, whereas the IL-12R binding assay identified 3 putative neutralizing IL-12R antibodies (Table 1). The immunoprecipitation assay measured the ability of the putative anti-IL-12R antibodies that are immobilized on a solid phase to capture the solubilized complex of ¹²⁵I-IL-12/IL-12R. To verify that the radioactivity immunoprecipitated by the immobilized antibody was present in the complex of ¹²⁵I-IL-12/IL-12R, the immunoprecipitated proteins were solubilized, separated by SDS-PAGE and visualized by autoradiography. The preparations of the ¹²⁵I-IL-12/IL-12R complexes solubilized from PHA-activated PBMC, Kit-225 and K6 cells were resolved into two major radioactive bands, 210-250 kDa and 75 kDa (Figure 8). The 210-250 kDa and 75 kDa complexes were identified as the ¹²⁵I-IL-12/IL-12R complex and ¹²⁵I-IL-I2 not complexed with the receptor, respectively (Figure 8). See also Chizzonite et al., J. Immunol. 148, 3117 (1992). The radioactive 75 kDa band visualized from the cell extracts co-migrated with ¹²⁵I-IL-12, indicating that it represented ¹²⁵I-IL-12 that bound but was not covalently crosslinked to IL-12R. The 210-250 kDa band was not a covalent crosslinked oligomer of ¹²⁵I-IL-12 because it is not produced when the crosslinking agent BS3 was added directly to ¹²⁵I-IL-12 (Figure 8).

Hybridoma cells secreting putative anti-IL-12R antibodies were then cloned by limiting dilution and screened by both the immunoprecipitation and inhibition of binding assays that identify non-neutralizing and neutralizing antibodies, respectively. During this cloning and screening process, hybridoma lines secreting putative neutralizing anti-IL-12R antibodies were not recovered, whereas non-neutralizing antibodies were recovered from both the original immunoprecipitation and inhibitory positive hybridomas. After this initial identification and cloning, a direct binding assay was used to determine if the non-neutralizing antibodies only bound to cells expressing IL-12R. This assay demonstrated that the non-neutralizing antibodies could be divided into 2 classes, those that bound only IL-12R positive human cells and those that bound to most human cells (data not shown). Representative antibodies from each class, 2-4E6 and 2C6, respectively, were produced in ascites fluid, purified by protein G affinity chromatography and extensively characterized.

For Figure 8, PHA-activated PBMC (PHA-PBMC), Kit-225 (Kit-225) and K6 (K6) cells (1 x 10⁷ cells/ml) were incubated with ¹²⁵I-IL-12 (100-500 pM) for 2 hrs at room temperature in the absence or presence of 25 nM unlabelled IL-12. Cells were then washed, affinity crosslinked with BS3 (0.4 mM final concentration) and a cell extract prepared as described. The cell extract was precipitated with wheat germ lectin bound to solid supports as described. The precipitated proteins were released by treatment with sample buffer and analyzed by SDS-PAGE and autoradiography on a 8.0% slab gel. The complex of ¹²⁵I-IL-12 crosslinked to the IL-12 receptor migrates as a single major band of approximately 210-250 kDa. The band migrating at 75 kDa is ¹²⁵I-IL-12 that was bound but not crosslinked to the IL-12 receptor. ¹²⁵I-IL-12 (IL-12) and ¹²⁵I-IL-12 that was treated with the BS3 crosslinker (IL-12/BS3) were electrophoresed in parallel lanes as markers for the migration of the 75 kDa IL-12 heterodimer and for any oligomers of IL-12 that may form with the BS3 crosslinker. The molecular sizes indicated in the margins were estimated from standards run in parallel lanes. Exposure time was 7 days.

**Table 1**

| INITIAL IDENTIFICATION OF HYBRIDOMAS SECRETING ANTI-IL-12 RECEPTOR ANTIBODIES: SPLENOCYTES FROM MICE #211-1 AND #211-2 | | | |
|---|---|---|---|
| HYBRIDOMA/ANTIBODY I.P. ASSAY¹ (cpm bound) | | | INHIBITION ASSAY² |
| IL-12R 2C6³ | | 1900 | - |
| 211-1 | 1A5 | 722 | - |
| | 4E6 | 840 | - |
| | 5C1 | 312 | + |
| 211-2 | 3B1 | 1323 | - |
| | 4A3 | 2172 | - |
| | 4D6 | 804 | - |
| | 5D5 | 877 | - |
| | 4A5 | 509 | + |
| | 4C6 | 456 | + |
| | 1D1 | 1395 | - |
| | 5E6 | 2043 | - |
| | 2-4E6 | 2836 | - |
| Control mAb 402 | | - | |

| | | | |
|---|---|---|---|
| ¹ I.P. assay measures the amount of ¹²⁵I-IL-12/IL-12R complex bound by the immobilized antibody. | | | |
| ² Inhibition assay measures whether the antibody can inhibit ¹²⁵I-IL-12 binding to PHA-activated PBMC. | | | |
| ³ IL-12R 2C6 is an antibody that both immunoprecipitates the ¹²⁵I-IL-12/IL-12R complex and binds to many IL-12R positive and negative human cells. This antibody probably recognizes a component closely associated with the IL-12R. | | | |

### Example 14

### Characteristics of Monoclonal Anti-IL-12R Antibody 2-4E6 Binding to Natural IL-12R

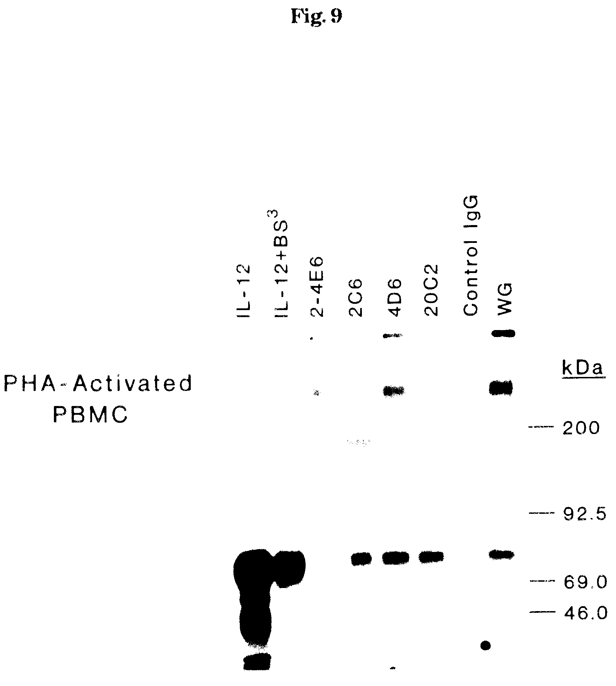

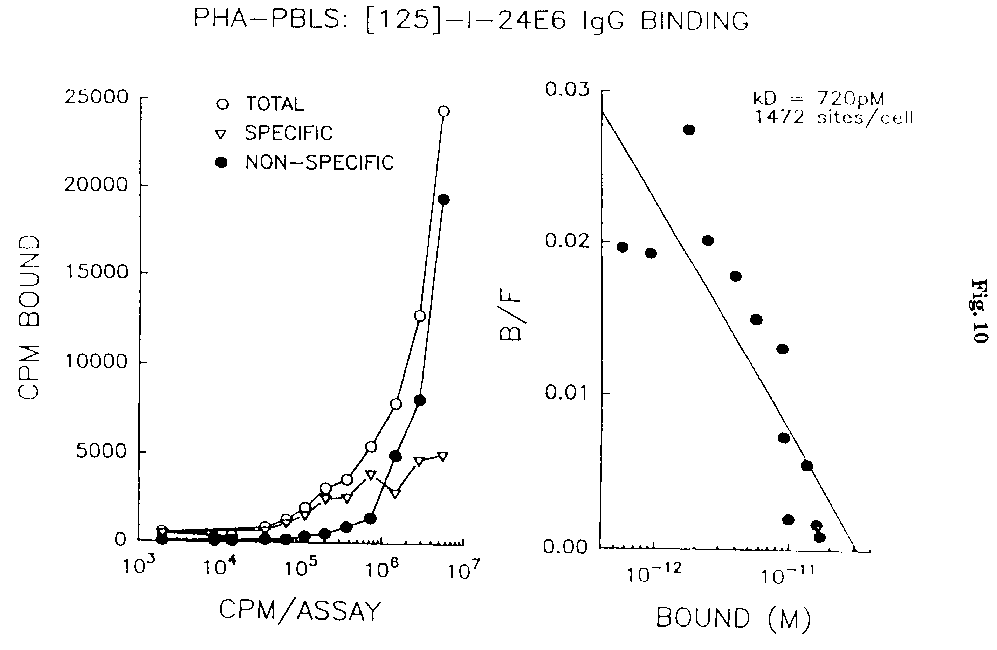

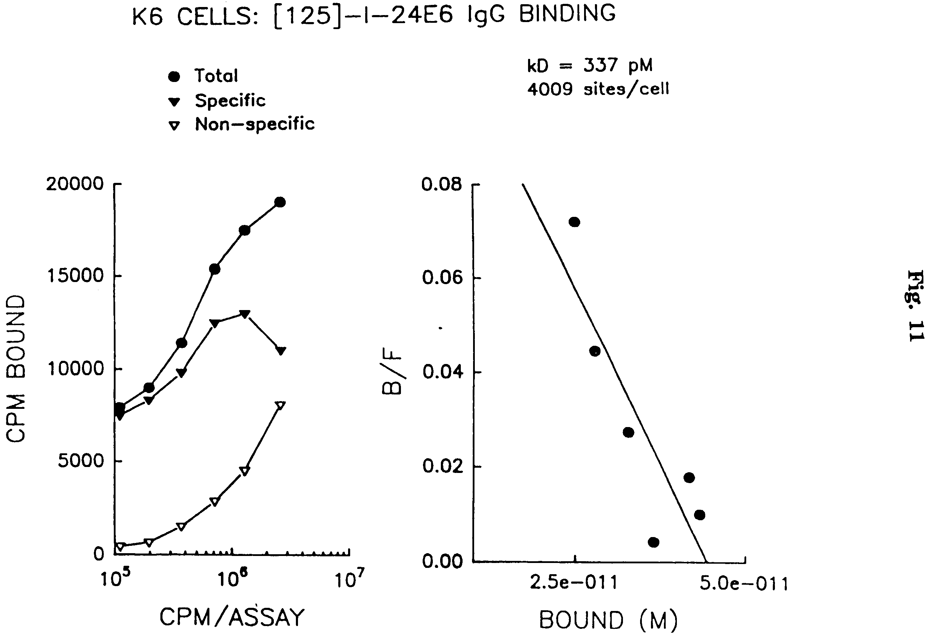

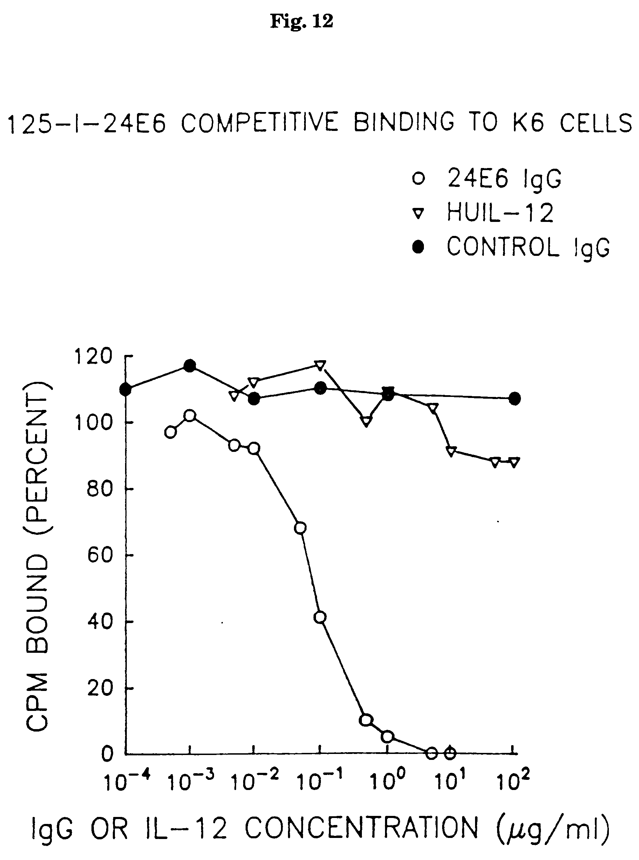

mAb 2-4E6 immunoprecipitates the ¹²⁵I-IL-12/IL-12R complex solubilized from PHA-activated human lymphoblasts, Kit-225 and K6 cells (Figure 9, data shown for PHA-activated PBMC), but does not block ¹²⁵I-IL-12 binding to IL-12R expressed on these cells. These data suggested that the 2-4E6 antibody was a non-inhibitory or non-neutralizing anti-IL-12R antibody. To confirm that 2-4E6 was an non-inhibitory antibody specific for the IL-12R, 2-4E6 was labelled with ¹²⁵I and direct binding assays were performed with IL-12R positive and negative cells. ¹²⁵I-2-4E6 binds to IL-12R bearing cells with an affinity that ranges from 337 pM to 904 pM and identifies between 1500 and 5000 binding sites per cell (PHA-activated PBMC, Figure 10; K6 cells, Figure 11). IL-12 does not block ¹²⁵I-2-4E6 from binding to PHA-activated PBMCs and confirms that 2-4E6 is a non-inhibitory/non-neutralizing antibody (Figure 12). ¹²⁵I-2-4E6 binds to other cells expressing IL-12R, such as Kit 225, and YT cells, but does not bind to IL-12R negative cells (non-activated human PBMC, MRC-5 fibroblasts and HL-60 cells (Table 2).

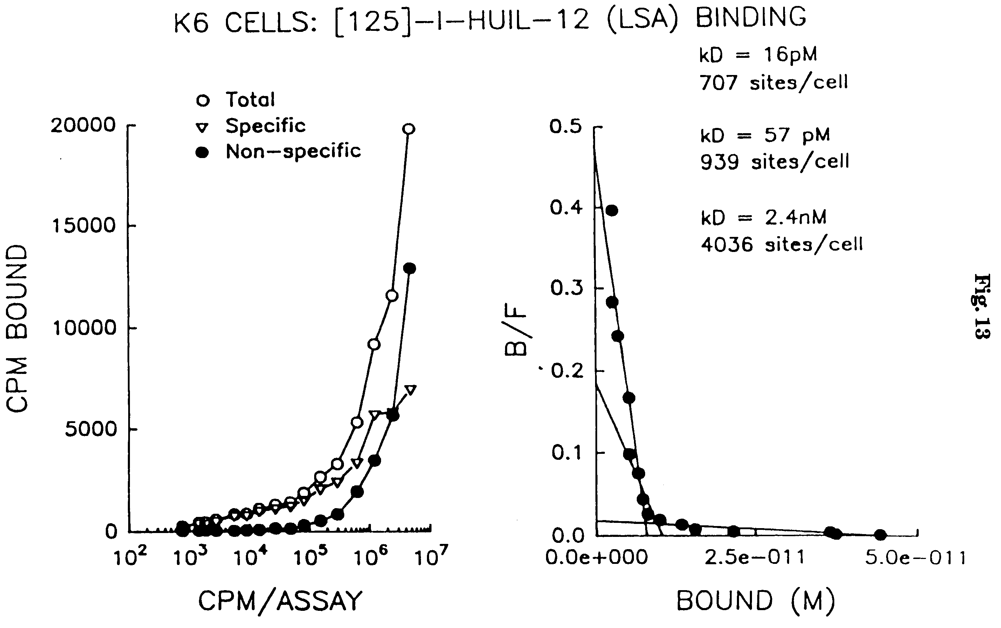

Equilibrium binding assays have demonstrated that ¹²⁵I-IL-12 identifies 3 separate binding sites on the surface of PHA-activated PBMCs, Kit-225 and K6 cells (Figure 13, data for K6 cells and Table 2). Analysis of this binding data by the method of Scatchard, supra shows these affinities are approximately 5-20 pM, 50-200 pM and 2-6 nM, respectively. The total number of ¹²⁵I-IL-12 binding sites per cell are approximately 1500 to 5000, which is in good agreement with the total number of binding sites identified by ¹²⁵I-2-4E6 (Table 2). The data also suggests that 2-4E6 recognizes the low affinity (2-5 nM) binding component of the IL-12 receptor in much the same manner that the anti-TAC antibody recognizes the low affinity component (p55 subunit) of the IL-2 receptor.

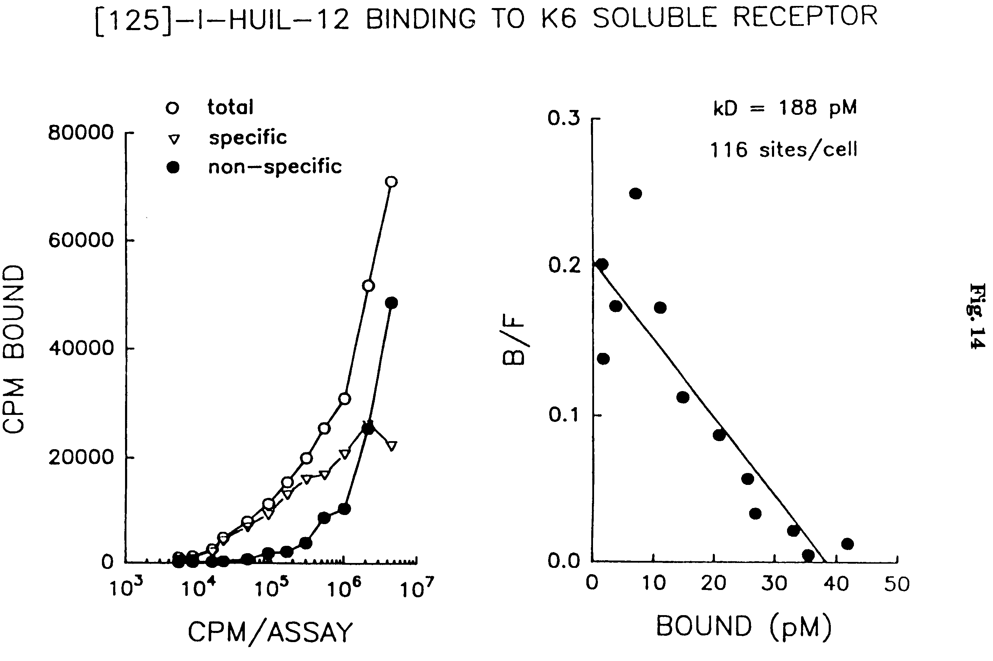

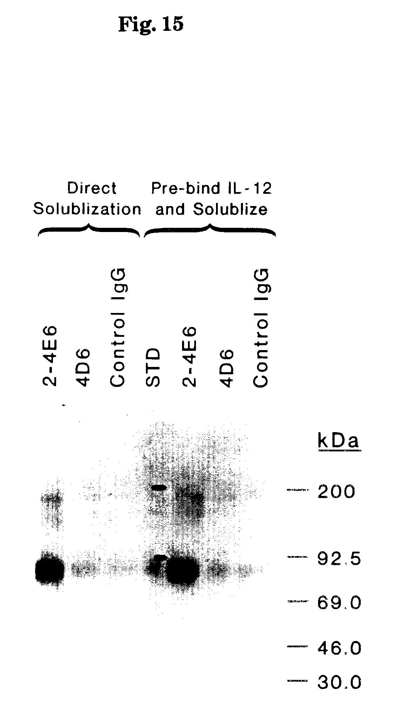

Since the data indicated that mAb 2-4E6 was a non-neutralizing antibody specific for the IL-12R, the molecular weight and ¹²⁵I-IL-12 binding characteristics of the protein(s) immunoprecipitated by mAb 2-4E6 from the surface of IL-12R positive cells was investigated. The steady state binding of ¹²⁵I-IL-12 to proteins immunoprecipitated by immobilized 2-4E6 from solubilized extracts of PHA-activated PBMCs, Kit-225 and K6 cells was saturable and specific (Figure 14, data for extracts from K6 cells). Transformation of the binding data by the method of Scatchard, revealed a single site with an apparent affinity of 188 pM. The proteins immunoprecipitated by 2-4E6 from the cell extracts were resolved by SDS-PAGE, transferred to nitrocellulose membrane and probed with ¹²⁵I-2-4E6 in a western blot. On the western blot, ¹²⁵I-2-4E6 binds to an approximately 90 kDa protein, that is only immunoprecipitated by 2-4E6 and not by an anti-IL-12 antibody or a control antibody (Figure 15, data shown for PHA-activated PBMCs). In summary, all the data demonstrated that mAb 2-4E6 bound a protein on the surface of IL-12R positive cells that was approximately 90 kDa and bound ¹²⁵I-IL-12 in a specific manner.

For Figure 9, soluble complexes of ¹²⁵I-IL-12/IL-12R were prepared from PHA-activiated human PBMC as detailed herein (see also Figure 8) and immunoprecipitated by immobilized antibodies, 2-4E6, 2C6, 4D6, 20C2 and control. The soluble complexes were also precipitated with wheat germ lectin immobilized on crosslinked agarose. The precipitated proteins were analyzed as described herein and in Figure 8. Antibodies 4D6 and 20C2 are non-neutralizing and neutralizing anti-IL-12 antibodies, respectively. 4D6 immunoprecipitates ¹²⁵I-IL-12/IL-12R complex and free ¹²⁵I-IL-12, whereas 20C2 only immunoprecipitates free ¹²⁵I-IL-12. Both 2-4E6 and 2C6 recognize the ¹²⁵I-IL-12/IL-12R complex. ¹²⁵I-IL-12 (IL-12) and ¹²⁵I-IL-12 that was treated with the BS3 crosslinked (IL-12/BS3) were electrophoresed in parallel lanes as markers for the migration of the 75 kDa IL-12 heterodimer and for any oligomers of IL-12 that may form with the BS3 crosslinker. The molecular sizes indicated in the margins were estimated from standards run in parallel lanes. Exposure time was 7 days.

For Figure 10, Lymphoblasts (1 x 10⁶ cells) were incubated for 2 hrs at room temperature with increasing concentrations of ¹²⁵I-2-4E6 in the absence (^{o}) or presence (·) of 25 nM unlabelled 2-4E6. Total (^{o}) and non-specific (·) cell bound radioactivity were determined as described. Specific binding of ¹²⁵I-2-4E6 ( ∇ ) was calculated by subtracting non-specific binding from total binding. The right hand panel shows analysis of the binding data according to the method of Scatchard as determined by Ligand computer program with a single-site model.

For Figure 11, K6 cells (1 x 10⁶ cells) were incubated for 2 hrs at room temperature with increasing concentrations of ¹²⁵I-2-4E6 in the absence (·) or presence ( ∇ ) of 25 nM unlabelled 2-4E6. Total (·) and non-specific ( ∇ ) cell bound radioactivity were determined as described. Specific binding of ¹²⁵I-2-4E6 ( 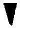 ) was calculated by subtracting non-specific binding from total binding. The right hand panel shows analysis of the binding data according to the method of Scatchard as determined by Ligand with a single-site model.

For Figure 12, The data are expressed as the amount of ¹²⁵I-2-4E6 bound [CPM BOUND (Percent)] to the cells in the presence of the indicated concentrations of unlabelled antibody or IL-12 when compared with the total specific binding in the absence of unlabelled competitor.

For Figure 13, K6 cells (1 x 10⁶ cells) were incubated for 2 hrs at room temperature with increasing concentrations of ¹²⁵I-IL-12 in the absence (^{o}) or presence (·) of 50 nM unlabelled IL-12. Total (^{o}) and non-specific (·) cell bound radioactivity were determined as described. Specific binding of ¹²⁵I-IL-12 ( ∇ ) was calculated by subtracting non-specific binding from total binding. The right hand panel shows analysis of the binding data according to the method of Scatchard as determined by Ligand with a single-site model.

For Figure 14, K6 cells 1.5 x 10⁸ cells/ml) were solubilized with 8 mM CHAPS extraction buffer and the cell extract (0.2 ml) was immunoprecipitated for 16 hrs at 4°C with mAb 2-4E6 immobilized on goat anti-mouse IgG coupled to agarose as described. Following this incubation, the beads were pelleted, washed and resuspended in IP buffer containing ¹²⁵I-IL-12 at concentrations ranging from 7 pM to 7.5 nM. The IL-12R immobilized on the 2-4E6 coated beads was incubated with ¹²⁵I-IL-12 for 2 hrs at RT and IL-12R bound radioactivity was determined in the presence of 50 nM unlabelled IL-12. The right hand panels show analysis of the binding data according to the method of Scatchard as determined by Ligand with a single-site model.

For Figure 15, PHA-activated PBMC (1 x 10⁸ cells/ml) were solubilized with 8 mM CHAPS extraction buffer and the cell extract (1 ml) was immunoprecipitated as described in Figure 13. Following this incubation, the beads were pelleted, washed and the bound proteins released by treatment with 0.1 M glycine pH 2.3. The released proteins were separated by non-reducing SDS/PAGE on 8% gels transferred to nitrocellulose membrane and probed with ¹²⁵I-2-4E6 as described. The molecular sizes indicated in the margins were estimated from molecular weight standards (Amersham Prestained High Molecular Weight Standards) run in parallel lanes. Exposure time was 7 days.

**Table 2**

| COMPARISON OF THE BINDING OF IL-12 AND 2-4C6 TO HUMAN CELLS EXPRESSING IL-12 RECEPTOR | | | | |
|---|---|---|---|---|
| CELL TYPE | IL-12 BINDING¹ | | 2-4E6 BINDING² | |
| Human Cells | K_{D} (nM) | Sites/cell | K_{D} (nM) | Sites/cell |
| non-activated human PBMC³ | none | detected | none | detected |
| PHA-PBMC (5-7 days)(3 sites) | 0.018 | 312 | 0.745 | 1472-2246 |
| | 0.084 | 501 | | |
| | 1.800 | 1406 | | |
| K6 cells (3 sites) | 0.016 | 707 | 0.489 | 3116-5259 |
| | 0.057 | 939 | | |
| | 2.400 | 4036 | | |
| Kit-225 (3 sites) | 0.023 | 100 | 0.594 | 1950 |
| | 0.210 | 250 | | |
| | 2.360 | 755 | | |
| YT cells (2 sites) | 0.006 | 24 | 0.904 | 4522 |
| | 0.109 | 117 | | |
| RAJI cells | none | detectable | 0.450 | 561 |
| MRC-5 | none | detectable | none | detectable |
| HL-60 | none | detectable | none | detectable |

| | | | | |
|---|---|---|---|---|
| ¹ Steady state ¹²⁵I-IL-12 binding assays. Apparent dissociation constant (K_{D}) and binding sites per cell have been calculated by transformation of the data by the method of Scatchard. | | | | |
| ² Steady state ¹²⁵I-2-4E6 binding assays. Data transformed by the method of Scatchard. | | | | |
| ³ Human peripheral blood mononuclear cells (PBMC) were activated with PHA as described in the methods (PHA-PBMC). | | | | |

### Example 15

### mAb 2-4E6 Binding To Human Recombinant IL-12R Expressed in COS Cells

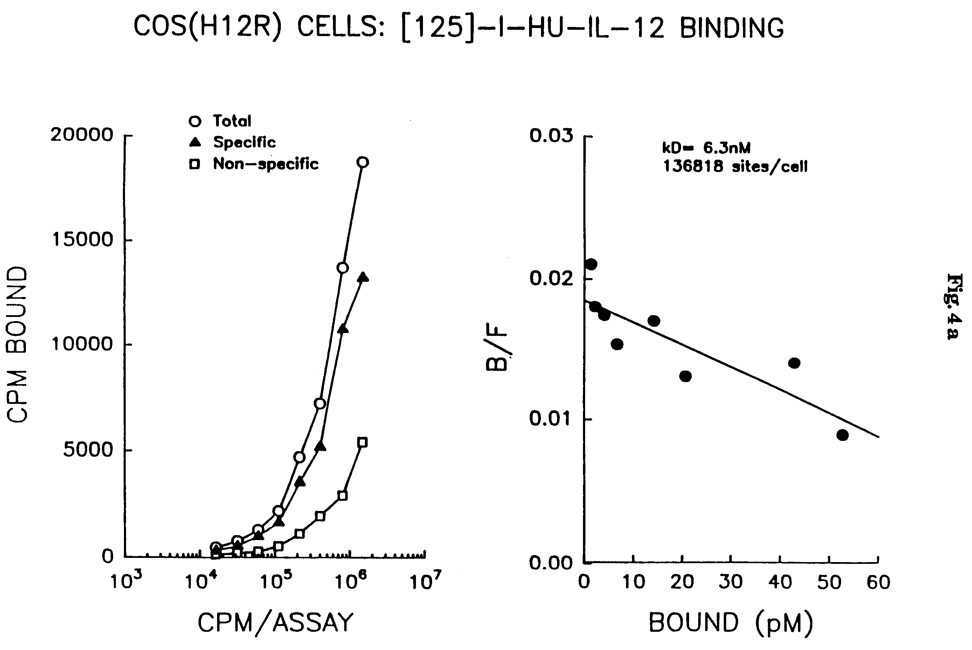

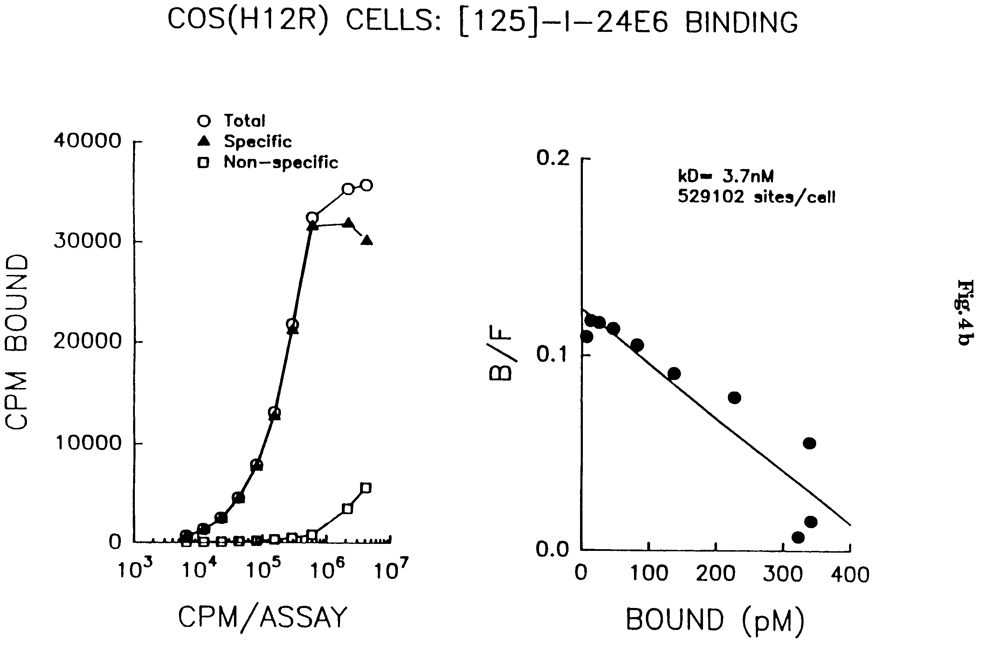

The characteristics of the protein bound by mAb 2-4E6 fulfilled standard criterion for an IL-12R and therefore 2-4E6 was used in an expression cloning strategy to isolate a cDNA coding for the human IL-12R. A cDNA coding for the human IL-12R was isolated by this method. The IL-12R cDNA was engineered in a mammalian cell expression vector, transfected into COS-7 (ATCC CRL 1651) cells and the specificity for binding of ¹²⁵I-IL-12 and ¹²⁵I-2-4E6 was determined. Steady state binding of ¹²⁵I-IL-12 to the rIL-12R expressing COS cells identifies a single binding site with an apparent affinity of 2-6 nM and approximately 150,000 sites/cell (Figure 4A). This low affinity IL-12 binding site corresponds to the low affinity site seen in the binding assays with human cells that naturally express IL-12R. The binding of 125I-2-4E6 to rIL-12R expressed in the COS cells is saturable and specific and identifies approximately 500,000 sites/cell (Figure 4B). COS cells transfected with an unrelated plasmid do not bind either ¹²⁵I-IL-12 or ¹²⁵I-2-4E6 (data not shown). These data demonstrated unequivocally that mAb 2-4E6 was specific for the low affinity component of the IL-12R.

For Figure 4A, COS cells were transfected with a plasmid expressing human rIL-12R as described. Three days later, transfected cells (1 x 10⁴ cells) were incubated for 2 hrs at room temperature with increasing concentration of ¹²⁵I-IL-12 in the absence ( ○ ) or presence ( □ ) of 50 nM unlabelled IL-12. Total ( ○ ) and non-specific ( □ ) cell bound radioactivity were determined as described. Specific binding of ¹²⁵I-IL-12 ( ▲ ) was calculated by subtracting non-specific binding from total binding. The right hand panel shows analysis of the binding data according to the method of Scatchard as determined by Ligand with a single-site model.

For Figure 4B, COS cells were transfected with a plasmid expressing human rIL-12R as described. Three days later, transfected cells (1 x 10⁴ cells) were incubated for 2 hrs at room temperature with increasing concentrations of ¹²⁵I-2-4E6 in the absence ( ○ ) or presence ( □ ) of 50 nM unlabelled 2-4E6. Total ( ○ ) and non-specific ( □ ) cell bound radioactivity were determined as described. Specific binding of ¹²⁵I-2-4E6 ( ▲ ) was calculated by subtracting non-specific binding from total binding. The right hand panel shows analysis of the binding data according to the method of Scatchard as determined by Ligand with a single-site model.

### Example 16

### Analysis of mAb 2-4E6 Binding to IL-12R Positive Human Cells by Fluorescence Activated Cell Sorting (FACS)

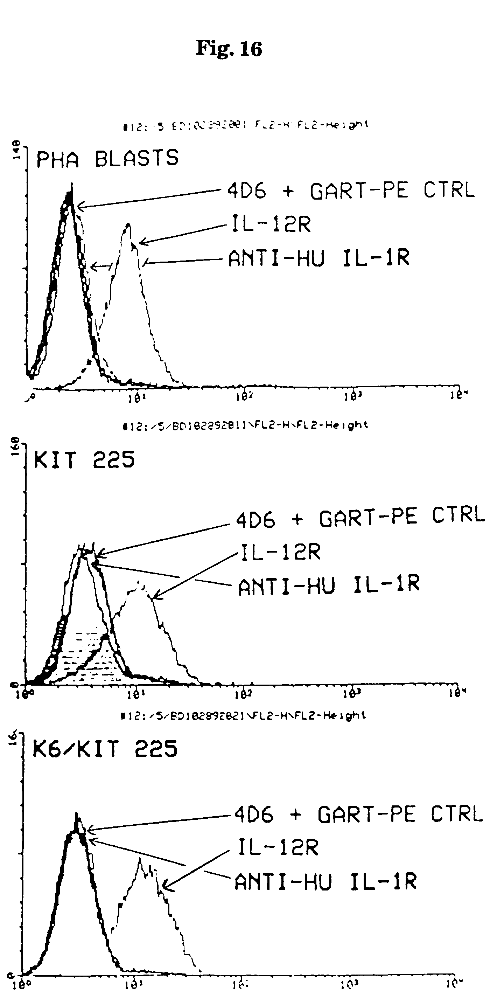

The expression level of IL-12R on human cells could be regulated depending on the activation state of the cells, the cell cycle or the type of environment from which the cells are isolated. Previous data had demonstrated that PHA activation of PBMC leads to a gradual rise in IL-12R expression, reaching a maximum at 3-4 days after activation and declining thereafter. Desai et al. (1992) J. Immunol., 148, 3125. To investigate the heterogeneity of IL-12R expression on PHA-activated PBMCs, Kit-225 and K6 cells, FACS analysis of IL-12R on these cells was determined with mAb 2-4E6 (Figure 16). The fluorescence intensity of binding of 2-4E6 was specific and indicated that these three cell types expressed approximately equal numbers of IL-12R. Interestingly, the FACS analysis indicated that the cell population was fairly homogenous and did not have one population expressing no or low numbers of IL-12R and a second population that expressed very high numbers of IL-12R.

For Figure 16, Day 4 PHA-activated lymphoblasts, Kit-225 and K6 cells were analyzed for IL-12R expressing cells by the indirect fluorescent antibody-labelling technique described. The figure depicts specific staining for IL-12R obtained in the presence of mAb 2-4E6 (IL-12R) and non-specific staining obtained in the presence of a control antibody specific for IL-1 receptor (anti-HuIL-1R), a control antibody specific for human IL-12 (4D6 + GART-PE CTRL) and the goat anti-mouse antibody conjugated with PE (GART-PE CTRL).

### Example 17

### Cell Culture

Peripheral blood mononuclear cells (PBMC) were isolated from blood collected from healthy donors. The blood was collected into heparinized syringes, diluted with an equal volume of Hank's balanced salt solution (HBSS) and layered over Ficoll-Hypaque. The tubes were spun at 2000 rpm for 20 minutes at room temperature. PBMC at the interface were collected and pelleted at 1500 rpm for 10 minutes through a 15 ml cushion of 20% sucrose in PBS. Pelleted PBMC were resuspended in tissue culture medium and washed twice in the same medium (RPMI 1640 plus 5 % serum). Finally, the cells were cultured at 0.5 - 1 x 10⁶ cells/ml in tissue culture medium plus 1 µg/ml PHA-P (Difco) for 3 days at 37°C in a 5% CO₂ atmosphere. Cells were split 1:1 in culture medium plus 50 U/ml rhuIL-2 (Roche) to yield >95% T-cells. The next day, these cells were used for assessing their responsiveness to IL-12, for radioligand (IL-12) binding assays and in flow cytometry assays for the detection of IL-12 receptors.

Flow cytometric detection of IL-12 receptors on such 4 day activated PHA blasts was performed as follows: the cells were washed twice in PBS and resuspended at 2 x 10⁶ cells/ml in PBS plus 2% fetal calf serum and 0.1% sodium azide. All the subsequent steps were carried out at 4°C. 1 x 10⁶ cells were incubated in 1 nM human IL-12 for 40 minutes. The cells were washed in FACS buffer and incubated with about 1 µg of biotinylated rat anti human p40 IL-12 subunit antibody 4D6 (Example 13) for 20 minutes. Cells were washed again and resuspended in 100 µl of a 5 µg/ml streptavidin-phycoerythritin conjugate (Fisher Biotech) for 15 minutes. The cells were then washed again before analysis on a FACSAN flow cytometer (Becton Dickinson).

### Example 18

### Extraction and characterization of RNA

PHA activated cells as described above were harvested at day 2-3 and total RNA was extracted using GuanidinIsothiocyanate/Phenol as described (P. Chomczynski and N. Sacchi (1987) Anal. Biochem., 162, 156). Poly A+ RNA was isolated from the total RNA by one batch adsorption to oligo dT latex beads as described (K. Kuribayashi et al (1988) Nucl. Acids Res. Symposium Series 19, 61). The mass yield of this purification was about 4%.

RNA blots were performed as follows. RNA was fractionated in 1.2 % agarose gels under denaturing conditions in the presence of 2.2M formaldehyde and subsequently transferred to nitrocellulose as described (Molecular Cloning, a Laboratory Manual, second edition, J. Sambrook, E.F. Fritsch, T. Maniatis, Cold Spring Harbour Laboratory Press 1989 (hereinafter "Molecular Cloning Manual)). The RNA blots were hybridized (7 x 10⁵ cpm/ml, 30 ml) with labelled probe in 5 x SSC (1X SSC = 0.15 M NaCl-0.015 M NaCitrate) - 50 % formamide - 5 x Denhardts solution (1 x Denhardts = 0.02% polyvinylpyrrolidone, 0.02 % Ficoll 400, 0.02% bovine serum albumin fraction V ) - 0.3 % SDS - 250 µg/ml denatured salmon sperm carrier DNA at 37°C overnight. The probe was generated by random-primer labelling gel-isolated insert from IL-12 receptor cDNA clone No. 5 as described in the above manual. The blots were first quickly rinsed at room temperature in 2 x SSC , then washed in 0.1 x SSC at 50°C for 30 minutes, dried and exposed to Kodak XAR film at -70°C for 3 days.

### Example 19

### cDNA Library

From the above polyA+ RNA, a cDNA library was established in the mammalian expression vector pEF-BOS as follows: 3 µg of polyA+ RNA were reverse transcribed into single stranded cDNA using RNaseH minus reverse transcriptase (GIBCO BRL Life Technologies Inc., P.O.Box 9418, Gaithersburg, MD 20898). The resulting mRNA-cDNA hybrids were converted into blunt ended double stranded cDNAs by established procedures (U.Gubler and A.Chua, in: Essential Molecular Biology Volume II, T.A. Brown, editor, pp. 39-56, IRL Press 1991). BstXI linkers (A. Aruffo and B. Seed, Proc. Natl. Acad. Sci. USA (1987) 84, 8573) were ligated to the resulting cDNAs and cDNA molecules > 800 base pairs (bp) were selected over a Sephacryl SF500 column. A Sephacryl SF 500 column (0.8 x 29 cm) was packed by gravity in 10 mM Tris-HCl pH 7.8 - 1 mM EDTA - 100 mM NaAcetate. BstXI linkered cDNA was applied to the column and 0.5 ml fractions were collected. A small aliquot of each fraction was run on a 1% agarose gel, the gel was dried down and the size distribution of the radioactive cDNA visualized by exposure of the gel to X-ray film. cDNA molecules larger than 800 bp were selected in this fashion, pooled, concentrated by ethanol precipitation and subsequently ligated to the cloning vector. The cloning vector was the plasmid pEF BOS (see reference supra) that had been cut with BstXI and purified over two consecutive gels. 300 ng of plasmid DNA were ligated to 30 ng of size selected cDNA from above in 60 µl of ligation buffer (50 mM Tris-HCl pH 7.8 - 10 mM MgCl₂ - 10 mM DTT - 1 mM rATP - 25 µg/ml bovine serum albumin) at 15°C overnight. The following day, the ligation reaction was extracted with phenol, 6 µg of mussel glycogen were added, and the nucleic acids were precipitated by ethanol. The precipitate was dissolved in water and the precipitation was repeated, followed by a wash with 80% ethanol. Finally, the pellet was dissolved in 6 µl of water and 1 µl aliquots were subsequently electroporated into *E. coli* strain DH-10B (BRL). By electroporating 5 parallel aliquots in this fashion, a library of about 10 million recombinants was generated for future use.

### Example 20

### Screening for IL-12 Receptor cDNAs by Panning

The basic principle of the panning method has been described in A. Aruffo and B. Seed, Proc. Natl. Acad. Sci. USA (1987) 84, 8573 as discussed below. Ten library aliquots each representing about 50,000 clones were plated on LB amp plates and were grown up overnight. The next day, the colonies from each pool were scraped off into a separate 50 ml aliquot of LB + amp and the cultures were grown for another two hours before plasmid DNA was extracted using QIAGEN plasmid kits. The ten separate DNA pools were then transfected into COS cells, using the DEAE dextran technique (2 million COS cells/9 cm diameter plate and 2.5 µg DNA) (Molecular Cloning Manual). 2 to 3 days later, the COS cells were detached from the plates using 0.5 mM EDTA/0.02% Na Azide in PBS and a single cell suspension was prepared for each pool. The monoclonal anti IL-12 receptor antibody 2-4E6 as discussed above was subsequently bound to the cells in suspension (10 µg/ml in PBS-0.5mM EDTA-0.02% Na Azide-5% FCS, 1 hour, on ice). The cell suspension was then spun through a layer of 2% Ficoll in the above buffer (tabletop centrifuge, 1000 rpm, 4 minutes) to eliminate the excess unbound antibody and the cells were gently resuspended in the same buffer. The cells from one pool were subsequently added to one bacterial petri dish (9 cm diameter) that had been coated with polyclonal goat anti mouse IgG (20 µg/ml in 50 mM Tris-HCl pH 9.5, RT/OVERNIGHT (ON)) and blocked with 1% BSA in PBS (37°C/1 hour). COS cells were panned in this way for 2 hours at RT. Nonadhering cells were then gently washed off with PBS and the remaining adherent cells in the dishes lysed by the addition of 0.8 ml of Hirt lysis solution (0.6% SDS-10 mM EDTA). After transferring to Eppendorf tubes, the lysates were made 1 M NaCl, incubated ON at +4°C and then spun at 15,000 rpm for 10 minutes in the cold. The supernatants were extracted with phenol once, 12 µg of mussel glycogen was added and the DNA precipitated twice by adding 0.5 volumes of 7.5 M NH₄OAc and 2.5 volumes of ethanol. The resulting DNA pellet was washed once with 80% ethanol, dried and taken up in 1 µl of distilled H₂0. The entire prep was then electroporated into *E. coli* strain DH-10B and the resulting colonies grown up ON. This represents one panning cycle. The ten library aliquots were panned each one separately for a total of three cycles.

From the last cycle of each pool, DNA was again extracted and this time transfected into COS cells plated on plastic one-chamber microscopic slides (2 slides per pool). 2-3 days after transfection, to one of the slides was bound labelled human IL-12 (10⁶ cpm/ml = 300 pM in RPMI 1640 plus 5% FCS for 2-3 hours at 4°C) and to the other slide labelled monoclonal Ab 2-4E6 (2 x 10⁶ cpm/ml = 1 nM in RPMI 1640 plus 5% FCS for 1 hour at RT). The slides were washed in PBS, fixed for 40 seconds in a cold mixture of methanol:acetone (7:3) and air dried. The slides were subsequently dipped in Kodak photographic emulsion NTB2, air dried and exposed in a light-tight container for 2-4 days at 4° C. They were developed in Kodak D10 developer according to the manufacturer's instructions and evaluated under a light microscope using a 10 to 40 fold bright field magnification. One of the ten pools, number 5, showed a large number of positive cells both for IL-12 and 2-4E6 binding. *E. coli* clones from this 3 x panned pool were subsequently picked into a microtiterplate (3 clones per well for a total of 288 clones). Pools representing the 8 rows and 12 columns from this plate were grown up and their plasmid DNA extracted. These 20 preps were transfected separately into COS cells on 12 well plates (10⁵ cells well, 4 wells per pool). 2-3 days after the transfection, labelled IL-12 was bound to the cells in two wells (total binding), whereas the other two wells per pool received labelled IL-12 and a 100 fold molar excess of cold IL-12 (= nonspecific binding). Wells were washed and the bound radioactivity eluted with 0.5 ml of 1% SDS and counted in a gamma counter. Two positive pools were identified in this manner, one representing column 1 and the other one representing row F from the microtiterplate. *E. coli* clones from well F1 must thus contain the IL-12 binding activity. Clones from that well were plated, and DNA from 10 single colonies was analyzed for plasmid insert size. 3 out of the 10 colonies showed an insert of about 2.1 kilobases in length, large enough to encode the IL-12 receptor. One of these clones was picked for further analysis.

### Example 21

### Characterization of IL-12 Receptor cDNAs

IL-12 receptor clone No. 5 was picked as described above and the plasmid DNA isolated. Gel isolated insert was sequenced on both strands using the ABI automated DNA sequencer in conjunction with a thermostable DNA polymerase and dye-labelled dideoxynucleotides as terminators.

Sequence alignments were run using the ALIGN program (M. O. Dayhoff et al. (1983) Meth. Enzymol. 91, 524) with the mutation data matrix, a break penalty of 6 and 100 random runs.

Cloned IL-12 receptor cDNAs were expressed in COS cells using either the DEAE dextran transfection or electroporation techniques (Molecular Cloning Manual). Binding assays with labelled IL-12 or labelled 2-4E6 antibody were run as described herein above under anti human IL-12 receptor antibody. The binding data were analyzed and Kd values were calculated according to Scatchard, using the Ligand program discussed herein above under anti human IL-12 receptor antibody. *In vivo* labelling (6 hours) of COS cells (3 x 10⁵ cells per 35 mm diameter tissue culture dish) with ³⁵S Cysteine was performed 3 days after transfection as described (Molecular Cloning Manual). Cells were washed in PBS and lysed in CHAPS lysis buffer (10 mM CHAPS - 300 mM NaCl - 50 mM Tris-HCl pH 7.4 - 2 mg/ml Iodoacetamide - 0.17 mg/ml PMSF), precleared by incubation with protein G Sepharose beads (50 µl packed beads per ml, Genex) and normal mouse serum (25 % final concentration) at 4°C overnight. The beads were spun out and labelled IL-12 receptor was specifically immunoprecipitated from the cleared lysates by adding 5 µg of 2-4E6 antibody per ml of sample. The antibody was diluted in PBS containing 1% bovine serum albumin and had been loaded onto 50 µl of packed beads for 2 - 3 hours at 4°C. Immunoprecipitation took place overnight at 4°C. The next day, the beads were washed 3-4 times in CHAPS lysis buffer before analysis on SDS-polyacrylamide gels as described (Molecular Cloning Manual).

### Example 22

### Lymphocyte Proliferation Assay

Lymphocyte proliferation assays to assess the ability of rat antisera to block cytokine-induced proliferation were performed as previously described (M.K. Gately, et al. (1992) Current Protocols in Immunology, vol. 1., J. E. Coligan, et al., eds., John Wiley & Sons, New York, NY, p. 6.16.1) with the following modifications. Aliquots of human PHA-activated PBMC (2x10⁴ per well) and of diluted rat sera were mixed in the wells of a 96-well plate and incubated at 37°C for 30 min. The cytokines (IL-12, IL-2 or IL-7) were then added to the wells, and the cultures were incubated for 48 h at 37°C. Following this, ³H-TdR was added to each well, and the cultures were harvested after an additional 7 h at 37°C. All values are the means of triplicates.

### Example 23

### Flow Cytometry

The titers of anti-COS cell antibodies in the various rat antisera were assessed by flow cytometry as follows. Untransfected COS cells (10⁶ cells/0.1 ml of Dulbecco's PBS containing 2% heat-inactivated FCS and 01 % sodium azide) were preincubated with 400 µg/ml normal rat IgG (Sigma, St. Louis, MO) for 15 min. on ice, and then with the indicated amount of rat serum for 30 min. on ice. The cells were washed and further incubated with 2 µg/ml FITC-conjugated F(ab')₂ mouse anti-rat IgG (Jackson ImmunoResearch Laboratories, Inc., West Grove, PA) for 30 min. on ice. The cells were again washed and then analyzed by flow cytometry using a FACScan (Becton-Dickinson, Mountain View, CA).

### Example 24

### Inhibition of IL-12-induced Lymphoblast Proliferation by Anti-IL-12R Antiserum

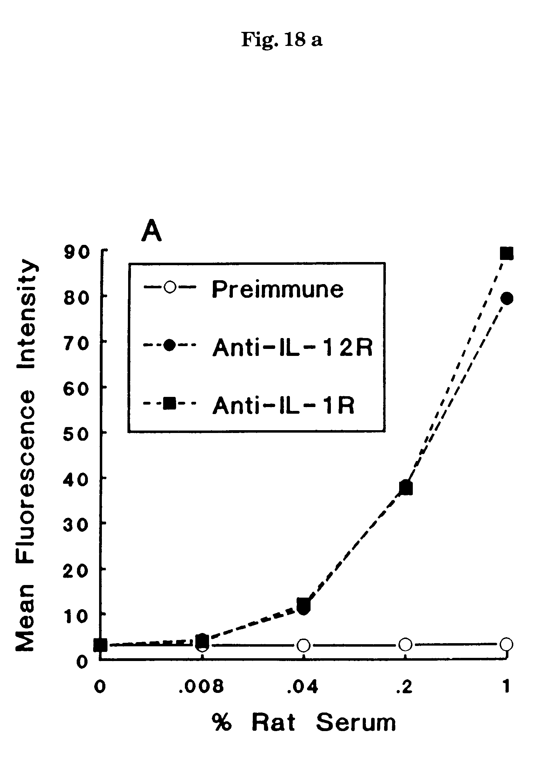

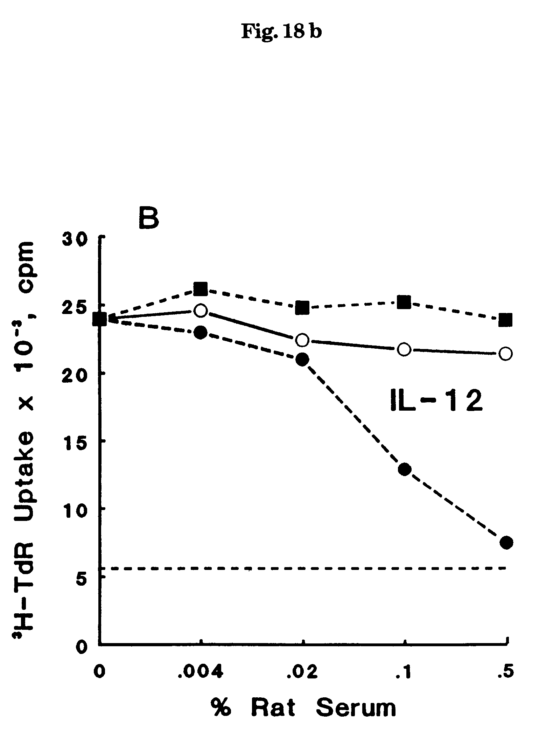

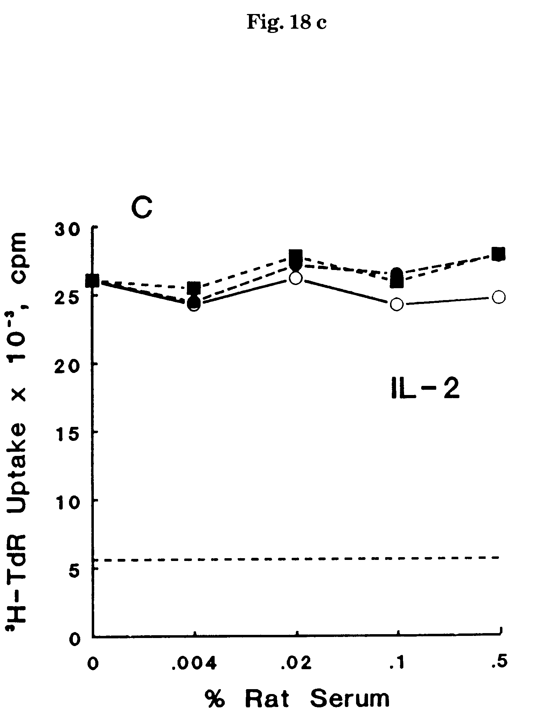

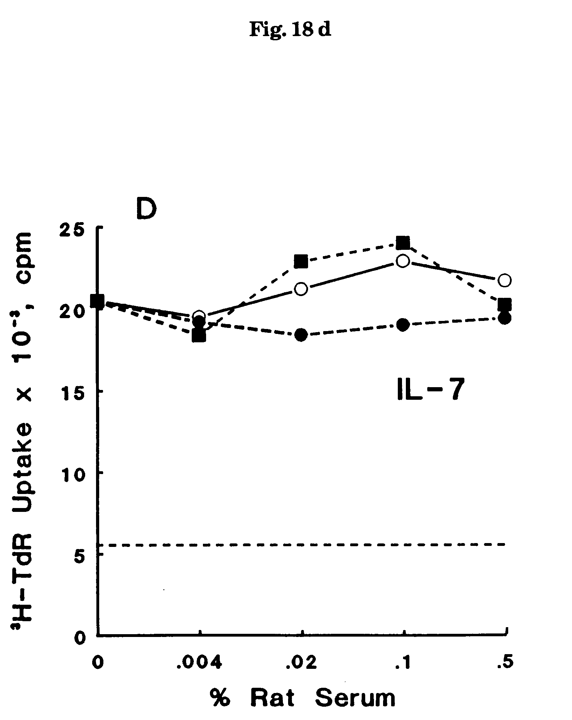

To determine whether the cloned IL-12R subunit plays an essential role in an IL-12-induced biologic response, we examined whether antiserum to the cloned IL-12R subunit could inhibit IL-12-induced proliferation of PHA-activated PBMC. This antiserum was produced by immunizing rats with 2-4E6-transfected COS cells and thus contained anti-COS cell antibodies as well as antibodies to the (putative) IL-12R subunit. For use as a control, we screened several other available rat antisera which had been prepared previously by immunization of rats with COS cells expressing proteins unrelated to the IL-12R. One such antiserum had been raised against COS cells transfected with human type II IL-1R and displayed a titer of anti-COS cell antibodies virtually identical to that of the anti-IL-12R antiserum (Figure 18A). We then compared the effects of the anti-IL-12R antiserum, the anti-IL-1R antiserum, and preimmune serum (from the rat used to prepare the anti-IL-12R) on lymphoblast proliferation induced by IL-12, IL-2, or IL-7. The concentrations of IL-12, IL-2, and IL-7 were 0.25 ng/ml, 1.25 ng/ml, and 0.4 ng/ml, respectively. These concentrations were chosen because they resulted in similar levels of ³H-TdR incorporation and were on the steep portion of the dose-response curves. In this experiment, the maximum levels of ³H-TdR incorporation in the presence of saturating amounts of cytokine were 38,820, 111,303, and 89,541 cpm for IL-12, IL-2, and IL-7, respectively. The level of ³H-TdR incorporation in the absence of any added cytokine is indicated by the horizontal dotted line. Two experiments were performed with essentially identical results, and one of these is illustrated in Figures 18B-D. The anti-IL-12R antiserum caused dose-dependent inhibition of IL-12-induced lymphoblast proliferation but had no effect on proliferation induced by IL-2 or IL-7. In contrast, neither the preimmune serum nor the anti-IL-1R antiserum inhibited lymphoblast proliferation induced by any of the three cytokines tested. These results strongly suggest that the cloned IL-12R subunit plays an essential role in mediating IL-12-induced proliferation of PHA-activated PBMC.

### Example 25

### Sequence Analysis of IL-12 Receptor cDNA Clones

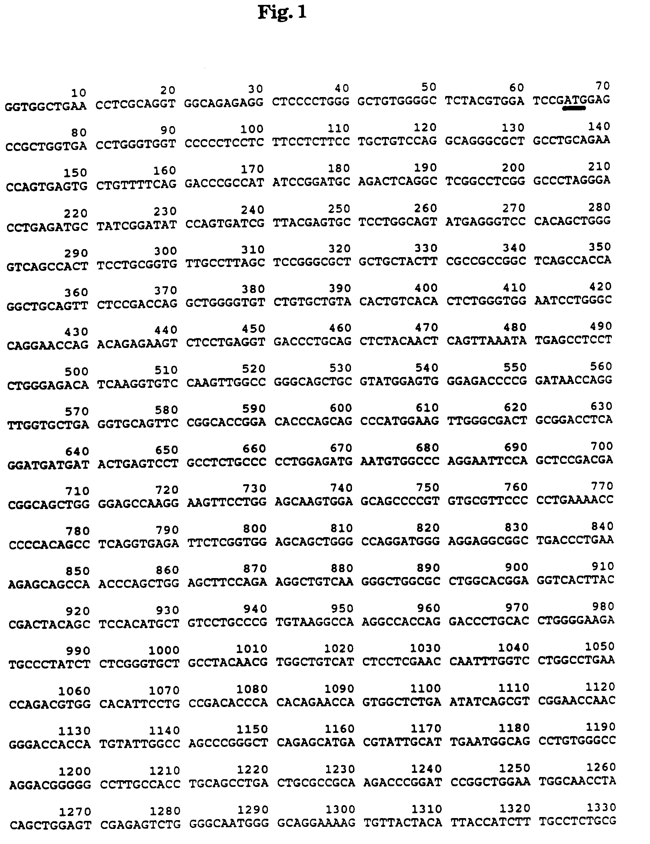

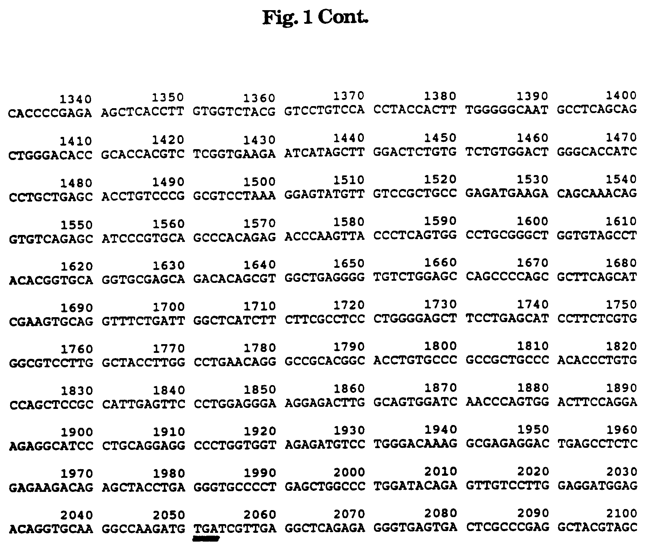

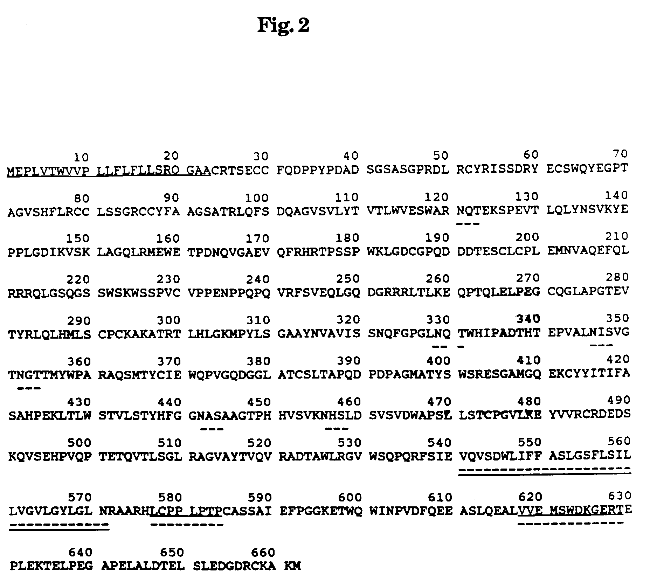

The DNA sequence for the IL-12 receptor cDNA insert from clone No. 5 is shown in Figure 1. The deduced amino acid sequence for the encoded receptor protein is shown in Figure 2. The IL-12 receptor protein is thus composed of 662 amino acids, and has the following features: N-terminal signal peptide, extracellular domain, transmembrane domain and cytoplasmic tail. The classical hydrophobic N-terminal signal peptide is predicted to be 20-24 amino acids in length. Signal peptide cleavage has been shown to occur mostly after the amino acids Ala, Ser, Gly, Cys, Thr, Gln (G. von Heijne, Nucl. Acids Res. (1986) 14, 4683). For the IL-12 receptor, the cleavage could thus take place after Gln20, Ala23 or Cys24 in the sequence shown in Figure 2, leaving a mature protein of 638 amino acids (calculated molecular weight = 70,426) based as cleavage at Cys24. The extracellular domain of the receptor is predicted to encompass the region from the C-terminus of the signal peptide to amino acid No. 540 in the sequence shown in Figure 2. Hydrophobicity analysis shows the area from amino acid No. 541 to 571 to be hydrophobic, as would be expected for a transmembrane anchor region. Charged transfer stop residues can be found at the N- as well as the C-terminus of this predicted transmembrane area. The extracellular domain of the receptor is thus 516 amino acids long and contains all the 6 predicted N-linked glycosylation sites. The cytoplasmic portion is 91 amino acids long (amino acid residue nos. 572 to 662) and contains 3 potential phosphorylation sites (S/TXXD/E) for casein kinase II.

The cDNA library was rescreened using the insert from clone No. 5 as the probe, and a second independent cDNA was isolated (clone No. 17). This cDNA contained an additional 202 bp of 3' untranslated region. The amino acid sequence (SEQ ID NO:3) deduced from this clone for the IL-12 receptor protein was almost completely identical to the sequence shown in Figure 2 (SEQ ID NO:2); however, a 13 bp deletion in the cDNA right before the stop codon changes the reading frame at the very C-terminus of the receptor and also gives rise to a protein that is 2 amino acids shorter (SEQ ID NO:3). Cycle sampling PCR was performed on uncloned cDNA using a pair of primers spanning the region that is expected to differ between the mRNAs representing clones 5 and 17. This analysis demonstrated that both transcripts coding for these two membrane-bound variants of the receptor subunit are present in the mRNA population isolated from PHA-activated PBMC at about equal levels (data not shown). The two transcripts are likely to arise from an alternate splicing event.

Further analysis of the amino acid sequence of the IL-12 receptor shows it to be a member of the cytokine receptor superfamily, by virtue of the sequence motifs [Cys52 --- Cys62SW] and [W222SKWS]. Comparing the IL-12 receptor sequence to all the members of the superfamily by running the ALIGN program shows that the IL-12 receptor has the highest homology to human gp130.

Sequence analysis of the IL-12 receptor extracellular domain demonstrated the presence of the hemopoietin receptor hallmark features: two pairs of conserved cysteine-residues and the WSXWS motif Further comparisons to the hemopoietin receptor superfamily showed the newly isolated IL-12 receptor component to be highly related to a subgroup of family members composed of gp130, G-CSF-receptor and LIF-receptor (Figure 3); align scores were 12.37 (IL-12R/gp130), 7.35 (IL-12R/G-CSF-R) and 6.31 (IL-12R/LIF-Rbeta). Similarities between the IL-12 receptor component and these three proteins extend beyond the hemopoietin receptor domain and include the area from the WSXWS motif to the transmembrane region (Figure 3). The extracellular portion of gp130 (M. Hibi et al. (1990) Cell, 63, 1149) was shown i) to contain the hemopoietin receptor superfamily domain and ii) to be also composed of 6 type III fibronectin repeats about 90 amino acids long (A.R. Kornblihtt, et al. (1985) EMBO J., 4, 1755; L. Patthy (1990) Cell 61, 13). Similarly, the extracellular domain of the IL-12 receptor can be subdivided into five such fibronectin type III repeats (residues 43-133, 143-236, 237-337, 338-444 and 445-540). The IL-12 receptor extracellular domain lacks the N-terminal Ig domain found in gp130 and therefore only accommodates 5 fibronectin type III repeats. Further sequence similarities between the IL-12 receptor, gp130, the G-CSF-receptor and the LIF-receptor can be found in the cytoplasmic regions (Figure 3). A PXXPXP motif within a box of 8 amino acids conserved between a number of different superfamily members and a second, 12 amino acid long conserved box were found to be important for signal transduction mediated by gp130 (M. Murakami et al. (1991) Proc. Natl. Acad. Sci. (USA), 88, 11349). Both those motifs are also found in conserved areas of the cytoplasmic part of the IL-12 receptor sequence (amino acid residues 577-584 and amino acid residues 618-629).

### Example 26

### Analysis of IL-12 Receptor mRNA Expression

RNA blots were performed using polyA+ RNA from cells known to respond to IL-12: PHA-stimulated PBMC and the CD4+ T-cell line Kit225. Two RNA transcripts about 3 Kb and 2.3 Kb in size are apparent when blots are probed with the full-length cDNA (Figure 6, lanes 1-3). Both RNAs are induced from undetectable or very low levels when PBMC are activated by PHA for 3 days (compare lanes 1 and 2); Kit225 cells express both transcripts constitutively (lane 3). Analysis by phosphoimager shows the level of the larger RNA to be about 3 to 5 fold higher than the level of the smaller RNA. Surprisingly, the smaller RNA does not hybridize to a probe derived from the cytoplasmic domain (lanes 4-6). This finding could indicate the presence of an RNA encoding i) a soluble IL-12 receptor protein, ii) a membrane bound IL-12 receptor devoid of a cytoplasmic region altogether or iii) an IL-12 receptor with a cytoplasmic sequence that is completely different from the ones present in clones 5 and 17. Elucidation of this question will have to await the isolation of a cDNA clone derived from the smaller RNA transcript.

### Example 27

### Characterization of Recombinant IL-12 Receptor

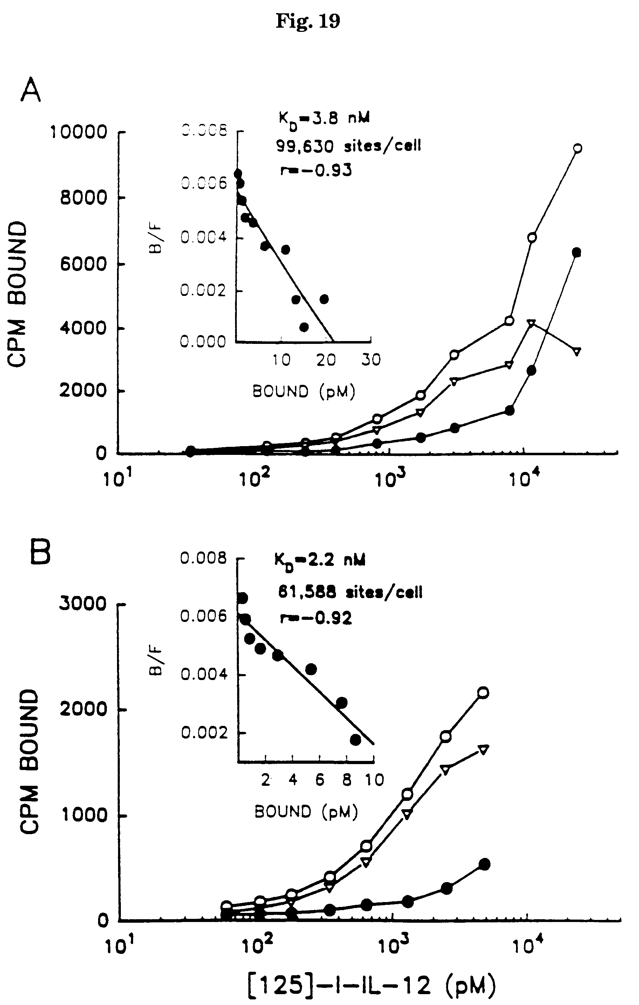

IL-12 receptor cDNA (clone number 5) (SEQ ID NO:1) was electroporated into COS cells and equilibrium binding of labelled human and murine IL-12 to the cells was performed and analyzed as described (R. Chizzonite, et al., 1992, J. Immunol., 148, 3117). Results are shown in Figure 19. Human and murine IL-12 bind to recombinant IL-12 receptor (SEQ ID NO:2) with a single affinity (K_{D}) of 3.4 ± 1.3 nM (n=7) and 2.1 ± 0.5 nM (n=4), respectively, which corresponds to the low affinity component of the functional IL-12 receptor on PHA-activated PBMC. After transformation by the method of Scatchard, the equilibrium binding data was best described by a single receptor site model as determined by the LIGAND program. The site numbers indicated in Figure 19 are calculated assuming that all cells are expressing receptors. IL-12 receptor protein expressed by clone number 17 (SEQ ID NO:3) gave similar results in these binding assays. SEQ ID NO:3 would also have the same regions as SEQ ID NO:2.

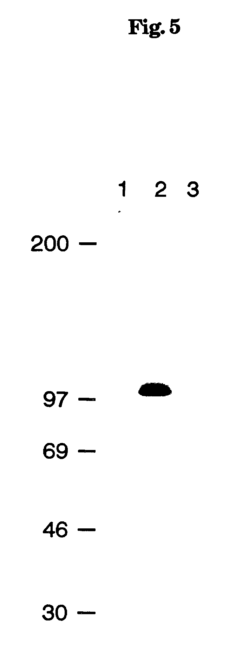

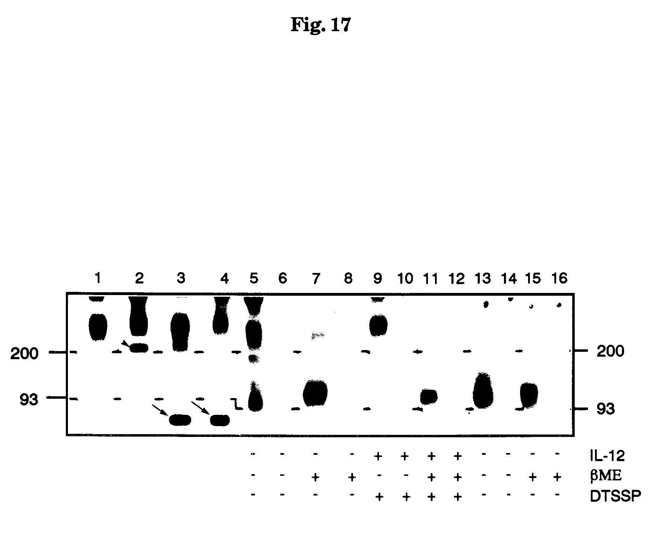

Metabolic labelling and immunoprecipitation of the IL-12 receptor subunit expressed in COS cells indicated its size to be about 100 KDa as determined by gel analysis under reducing conditions (Figure 5). To analyze the size of the receptor at the cell surface, affinity crosslinking studies were performed. Unless otherwise stated, characterization of the IL-12 receptor protein was done on SEQ ID NO:2. Crosslinking of 0.2 nM ¹²⁵I-labelled IL-12 to either transfected COS cells, PHA-activated PBMC or K6 cells gave rise to complexes that migrate at >200 KDa (Figure 17, lanes 1,3 and 4; arrow indicates uncrosslinked IL-12). Crosslinking at 2 nM ¹²⁵I-IL-12 (a concentration equivalent to the K_{D}) gave identical results (not shown). The size of a complex composed of one receptor subunit and one IL-12 ligand is expected to be about 175 KDa. However, Figure 17 shows that the 175 KDa complex is present only at very low levels, if at all. Since the 150 kDA Ig and the 200 KDa markers are not separated on the gel system used, the 175 KDa IL-12/IL-12 receptor complex is expected to comigrate with them. For comparison, lane 2 shows transfected COS cells crosslinked to labelled 2-4E6 antibody (arrowhead = uncrosslinked 2-4E6). Crosslinking labelled IL-12 to i) cells that do not bind IL-12 (e.g. Raji cells), ii) mock-transfected COS cells or iii) transfected COS cells in the presence of an excess of cold IL-12 did not yield any products (not shown). For Figure 17, labelled IL-12 (0.2 nM) was bound and crosslinked with BS3 (0.4 mM) to transfected COS cells (lane 1), PHA-activated PBMC (lane 3) or K6 cells (lane 4). Labelled 2-4E6 antibody (0.5 nM) was bound and crosslinked with BS3 (0.4 mM) to transfected COS cells (lane 2). Anti-IL-12 receptor antibody 2-4E6 (lanes 5,7), anti-IL12 antibody 4D6 (lanes 9,11) and control antibody (lanes 6,8,10,12,) were used. Antibody 2-4E6 (lanes 13,15) and control antibody (lanes 14,16) were used.

Since crosslinking of labelled IL-12 to IL-12 receptor gave rise to products that are larger than what is expected for a complex of one receptor and one IL-12 ligand but whose size is difficult to estimate, cell surface labelling and immunoprecipitation experiments of transfected COS cells were performed. Samples were analyzed under reducing and nonreducing conditions (Figure 17, lanes 5-12). The results can be summarized as follows: i) transfected COS cells express the IL-12 receptor subunit both as monomers and as a second, larger product that could be dimers or oligomers. Both these products are present at about equal levels (lane 5); ii) the dimerization/oligomerization does not depend on IL-12 binding. If IL-12 is prebound to the cells, the resulting banding pattern does not change (not shown); and iii) The dimers/oligomers can be converted to the monomers by reduction and must therefore be disulfide-bonded (lane 7). The data from the crosslinking and surface labelling experiments thus suggested that only the dimeric/oligomeric receptor subunit form binds IL-12 with the 3 nM affinity measured on transfected COS cells. This possibility was further investigated as follows. Complexes produced by binding unlabelled IL-12 to ¹²⁵I-surface labelled COS cells and crosslinking with a cleavable crosslinker were immunoprecipitated by an anti-IL-12 antibody and analyzed under non-reducing and reducing conditions (Figure 17, lanes 9-12). The anti-IL-12 antibody only precipitated a complex corresponding to IL-12 bound to the dimer/oligomer but not the monomer of the IL-12 receptor subunit (lane 9). Analysis of this complex under reducing conditions identified a labelled protein that co-migrated with the IL-12 receptor monomer (lane 11). Experiments with a murine CTLL cell transfectant stably expressing the IL-12 receptor subunit lend further support to our findings. These cells express about 3000 to 5000 receptor subunits per cell, as measured by 2-4E6 antibody binding; however, the cells bind IL-12 very inefficiently, with an estimated Kd of 50 nM or greater (not shown). The results from surface labelling and immunoprecipitation experiments with the CTLL transfectants clearly indicate that they only express IL-12 receptor subunit monomers (Figure 17, lanes 13-16). Taken together, the data support the hypothesis that only the receptor subunit dimers/oligomers bind IL-12 with the low affinity (3 nM) measured on transfected COS cells.

## Claims

1. A DNA molecule coding for a low affinity Interleukin-12 receptor or a functional derivative thereof which binds specifically to Interleukin-12.

2. A DNA molecule of claim 1 coding for a human low affinity Interleukin-12 receptor or a functional derivative thereof which binds specifically to Interleukin-12.

3. A DNA molecule of claims 1-2 having the sequence SEQ ID NO:1 or a degenerate variant thereof.

4. A DNA molecule of claims 1-3 which codes for the amino acid sequence SEQ ID NO:2 or SEQ ID NO:3 or portions thereof.

5. A DNA molecule of claims 1-4 which codes for a soluble, truncated Interleukin-12 receptor protein.

6. A vector comprising a DNA molecule as claimed in any of claims 1-5.

7. A host cell which has been transformed by a vector of claim 6.

8. A low affinity Interleukin-12 receptor protein or a functional derivative thereof which binds specifically to Interleukin-12.

9. A low affinity Interleukin-12 receptor protein encoded by a DNA molecule of claims 1-5.

10. A low affinity Interleukin-12 receptor protein of claims 8-9 wherein the Interleukin receptor has a K_{D} of about 2 to about 10 nM, preferably a K_{D} of about 2 to about 5 nM.

11. A low affinity Interleukin-12 receptor protein of claims 8-10 having an apparent molecular weight of about 100 kDa determined by gel electrophoresis under reducing conditions.

12. A low affinity Interleukin-12 receptor protein of claims 8-11 which is a dimer or oligomer of the 100 kD receptor subunit.

13. A low affinity Interleukin-12 receptor protein of claims 8-12 which corresponds to the low affinity component of the functional Interleukin-12 receptor on PHA-activated PBMC.

14. An immunoglobulin capable of binding selectively to an Interleukin-12 receptor protein as defined in any one of claims 8-13.

15. An immunoglobulin as claimed in claim 14, which is in the form of an anti-sera composition or in the form of a monoclonal antibody.

16. The immunoglobulin of claim 14-15 which is capable of inhibiting the binding of Interleukin-12 to the Interleukin-12 receptor and is capable of neutralizing IL-12 bioactivity by binding to the IL-12 receptor.

17. The immunoglobulin of 14-15 which binds to the Interleukin-12 receptor but which is not capable of inhibiting the binding of Interleukin-12 to the Interleukin-12 receptor and is not capable of neutralizing Interleukin-12 bioactivity by binding to human IL-12 receptor.

18. A protein as claimed in any one of claims 8-13 for the binding or scavenge of Interleukin-12.

19. An immunoglobulin as claimed in any one of claims 14-17 for neutralising and/or inhibiting Interleukin-12 bioactivity.

20. A pharmaceutical composition comprising a low affinity Interleukin-12 receptor protein as defined in any one of claims 8-13 and a suitable diluent or carrier.

21. A pharmaceutical composition comprising an Interleukin-12 receptor protein as claimed in claim 20 containing one or more other cytokine receptor antagonists.

22. A pharmaceutical composition comprising an immunoglobulin as claimed in claim 14-17 and a suitable diluent or carrier.

23. A composition comprising human cells activated to express Interleukin-12 receptor bound with an immunoglobulin as claimed in claim 14-17, which immunoglobulin is labelled with a detectable label.

24. Use of an Interleukin- 12 receptor according to any one of claims 8-13 for the preparation of a pharmaceutical composition.

25. Use of an Interleukin-12 receptor according to claim 24 for the preparation of a pharmaceutical composition for treatment of diseases or conditions caused by an immune response to alloantigen and in the treatment of autoimmune dysfunction.

26. Use of an Interleukin-12 receptor protein as claimed in any one of claims 8-13 to bind or scavenge Interleukin-12.

27. Use of an immunoglobulin as claimed in claim 14-17 for the preparation of a pharmaceutical composition.

28. A process for the detection of an Interleukin-12 receptor which comprises isolating cells from a subject wich express the Interleukin-12 receptor, contatcing a sample of said cells with a detectable immunoglobulin capable of selectively binding to the Interleukin-12 receptor , incubating said cells under conditions which allow the detectable immunoglobulin to bind to the Interleukin-12 receptor, and detecting the binding of said cells to said immunoglobulin.

29. A process of claim 28 wherein the immunoglobulin is covalently bound to a solid resin and/or said immunoglobulin is labelled with a detectable label.

30. A process for the detection of soluble Interleukin-12 receptor which assay comprises capturing said receptor with an immunoglobulin capable of selectively binding to the IL-12 receptor and carrying out a binding assay with labelled Interleukin-12.

31. A method for determining an immune system abnormality in a subject which comprises determining the number of T cells, NK cells, or B cells in a sample from the subject, contacting the sample with an immunoglobulin capable of forming a complex with the human IL-12 receptor so as to form a cellular complex between the human IL-12 receptor and the immunoglobulin, determining the percentages of the T cells, NK cells, or B cells in the sample which have the human IL-12 receptor, comparing the percentages of said cells with the percentages of such cells from a normal subject who does not have the immune system abnormality, a difference in the percentages of the cells so determined being indicative of the immune system abnormality.


SEQUENCE LISTING

(1) GENERAL INFORMATION:

(i) APPLICANT:
(A) NAME: F. HOFFMANN-LA ROCHE AG
(B) STREET: Grenzacherstrasse 124
(C) CITY: Basle
(D) STATE: BS
(E) COUNTRY: Switzerland
(F) POSTAL CODE (ZIP): CH-4002
(G) TELEPHONE: 061 - 688 51 08
(H) TELEFAX: 061 - 688 13 95
(I) TELEX: 962292/965542 hlr ch

(ii) TITLE OF INVENTION: INTERLEUKIN-12 RECEPTOR

(iii) NUMBER OF SEQUENCES: 3

(iv) COMPUTER READABLE FORM:
(A) MEDIUM TYPE: Floppy disk
(B) COMPUTER: Apple Macintosh
(C) OPERATING SYSTEM: System 7.1 (Mac)
(D) SOFTWARE: Microsoft Word 5.0

(2) INFORMATION FOR SEQ ID NO:1:

(i) SEQUENCE CHARACTERISTICS:
(A) LENGTH: 2104 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(vi) ORIGINAL SOURCE:
(A) ORGANISM: Homo sapiens
(G) CELL TYPE: human T-cells

(vii) IMMEDIATE SOURCE:
(A) LIBRARY: library 3 day PHA/pEF-BOS
(B) CLONE: human interleukin-12 receptor clone #5

(ix) FEATURE:
(A) NAME/KEY: CDS
(B) LOCATION: 65..2050

(xi) SEQUENCE DESCRIPTION: SEQ ID NO:1:
GGTGGCTGAA CCTCGCAGGT GGCAGAGAGG CTCCCCTGGG GCTGTGGGGC TCTACGTGGA 60
TCCG ATG GAG CCG CTG GTG ACC TGG GTG GTC CCC CTC CTC TTC CTC TTC 109
CTG CTG TCC AGG CAG GGC GCT GCC TGC AGA ACC AGT GAG TGC TGT TTT 157
CAG GAC CCG CCA TAT CCG GAT GCA GAC TCA GGC TCG GCC TCG GGC CCT 205
AGG GAC CTG AGA TGC TAT CGG ATA TCC AGT GAT CGT TAC GAG TGC TCC 253
TGG CAG TAT GAG GGT CCC ACA GCT GGG GTC AGC CAC TTC CTG CGG TGT 301
TGC CTT AGC TCC GGG CGC TGC TGC TAC TTC GCC GCC GGC TCA GCC ACC 349
AGG CTG CAG TTC TCC GAC CAG GCT GGG GTG TCT GTG CTG TAC ACT GTC 397
ACA CTC TGG GTG GAA TCC TGG GCC AGG AAC CAG ACA GAG AAG TCT CCT 445
GAG GTG ACC CTG CAG CTC TAC AAC TCA GTT AAA TAT GAG CCT CCT CTG 493
GGA GAC ATC AAG GTG TCC AAG TTG GCC GGG CAG CTG CGT ATG GAG TGG 541
GAG ACC CCG GAT AAC CAG GTT GGT GCT GAG GTG CAG TTC CGG CAC CGG 589
ACA CCC AGC AGC CCA TGG AAG TTG GGC GAC TGC GGA CCT CAG GAT GAT 637
GAT ACT GAG TCC TGC CTC TGC CCC CTG GAG ATG AAT GTG GCC CAG GAA 685
TTC CAG CTC CGA CGA CGG CAG CTG GGG AGC CAA GGA AGT TCC TGG AGC 733
AAG TGG AGC AGC CCC GTG TGC GTT CCC CCT GAA AAC CCC CCA CAG CCT 781
CAG GTG AGA TTC TCG GTG GAG CAG CTG GGC CAG GAT GGG AGG AGG CGG 829
CTG ACC CTG AAA GAG CAG CCA ACC CAG CTG GAG CTT CCA GAA GGC TGT 877
CAA GGG CTG GCG CCT GGC ACG GAG GTC ACT TAC CGA CTA CAG CTC CAC 925
ATG CTG TCC TGC CCG TGT AAG GCC AAG GCC ACC AGG ACC CTG CAC CTG 973
GGG AAG ATG CCC TAT CTC TCG GGT GCT GCC TAC AAC GTG GCT GTC ATC 1021
TCC TCG AAC CAA TTT GGT CCT GGC CTG AAC CAG ACG TGG CAC ATT CCT 1069
GCC GAC ACC CAC ACA GAA CCA GTG GCT CTG AAT ATC AGC GTC GGA ACC 1117
AAC GGG ACC ACC ATG TAT TGG CCA GCC CGG GCT CAG AGC ATG ACG TAT 1165
TGC ATT GAA TGG CAG CCT GTG GGC CAG GAC GGG GGC CTT GCC ACC TGC 1213
AGC CTG ACT GCG CCG CAA GAC CCG GAT CCG GCT GGA ATG GCA ACC TAC 1261
AGC TGG AGT CGA GAG TCT GGG GCA ATG GGG CAG GAA AAG TGT TAC TAC 1309
ATT ACC ATC TTT GCC TCT GCG CAC CCC GAG AAG CTC ACC TTG TGG TCT 1357
ACG GTC CTG TCC ACC TAC CAC TTT GGG GGC AAT GCC TCA GCA GCT GGG 1405
ACA CCG CAC CAC GTC TCG GTG AAG AAT CAT AGC TTG GAC TCT GTG TCT 1453
GTG GAC TGG GCA CCA TCC CTG CTG AGC ACC TGT CCC GGC GTC CTA AAG 1501
GAG TAT GTT GTC CGC TGC CGA GAT GAA GAC AGC AAA CAG GTG TCA GAG 1549
CAT CCC GTG CAG CCC ACA GAG ACC CAA GTT ACC CTC AGT GGC CTG CGG 1597
GCT GGT GTA GCC TAC ACG GTG CAG GTG CGA GCA GAC ACA GCG TGG CTG 1645
AGG GGT GTC TGG AGC CAG CCC CAG CGC TTC AGC ATC GAA GTG CAG GTT 1693
TCT GAT TGG CTC ATC TTC TTC GCC TCC CTG GGG AGC TTC CTG AGC ATC 1741
CTT CTC GTG GGC GTC CTT GGC TAC CTT GGC CTG AAC AGG GCC GCA CGG 1789
CAC CTG TGC CCG CCG CTG CCC ACA CCC TGT GCC AGC TCC GCC ATT GAG 1837
TTC CCT GGA GGG AAG GAG ACT TGG CAG TGG ATC AAC CCA GTG GAC TTC 1885
CAG GAA GAG GCA TCC CTG CAG GAG GCC CTG GTG GTA GAG ATG TCC TGG 1933
GAC AAA GGC GAG AGG ACT GAG CCT CTC GAG AAG ACA GAG CTA CCT GAG 1981
GGT GCC CCT GAG CTG GCC CTG GAT ACA GAG TTG TCC TTG GAG GAT GGA 2029
GAC AGG TGC AAG GCC AAG ATG TGATCGTTGA GGCTCAGAGA GGGTGAGTGA 2080
CTCGCCCGAG GCTACGTAGC CTTT 2104

(2) INFORMATION FOR SEQ ID NO:2:

(i) SEQUENCE CHARACTERISTICS:
(A) LENGTH: 662 amino acids
(B) TYPE: amino acid
(D) TOPOLOGY: linear

(ii) MOLECULE TYPE: protein

(ix) FEATURE:
(A) NAME/KEY: Region
(B) LOCATION: 1..23
(D) OTHER INFORMATION: /note= "N-terminal signal peptide
(1..20 or 23 or 24)"

(ix) FEATURE:
(A) NAME/KEY: Region
(B) LOCATION: 541..570
(D) OTHER INFORMATION: /note= "transmembrane region"

(ix) FEATURE:
(A) NAME/KEY: Region
(B) LOCATION: 571..662
(D) OTHER INFORMATION: /note= "cytoplasmic tail region"

(ix) FEATURE:
(A) NAME/KEY: Region
(B) LOCATION: 577..584
(D) OTHER INFORMATION: /note= "conserved area of cytoplasmic tail
region"

(ix) FEATURE:
(A) NAME/KEY: Region
(B) LOCATION: 618..629
(D) OTHER INFORMATION: /note= "conserved area of cytoplasmic tail
region"

(ix) FEATURE:
(A) NAME/KEY: Region
(B) LOCATION: 52..64
(D) OTHER INFORMATION: /note= "sequence motif of cytokine
receptor superfamily Cys52..Cys62SW"

(ix) FEATURE:
(A) NAME/KEY: Region
(B) LOCATION: 222..226
(D) OTHER INFORMATION: /note= "cytokine receptor
superfamily motif (W222SKWS)"

(ix) FEATURE:
(A) NAME/KEY: Region
(B) LOCATION: 121..123
(D) OTHER INFORMATION: /note= "N-linked glycosylation
site"

(ix) FEATURE:
(A) NAME/KEY: Region
(B) LOCATION: 329..331
(D) OTHER INFORMATION: /note= "N-linked glycosylation
site"

(ix) FEATURE:
(A) NAME/KEY: Region
(B) LOCATION: 346..348
(D) OTHER INFORMATION: /note= "N-linked glycosylation
site"

(ix) FEATURE:
(A) NAME/KEY: Region
(B) LOCATION: 352..354
(D) OTHER INFORMATION: /note= "N-linked glycosylation
site"

(ix) FEATURE:
(A) NAME/KEY: Region
(B) LOCATION: 442..444
(D) OTHER INFORMATION: /note= "N-linked glycosylation
site"

(ix) FEATURE:
(A) NAME/KEY: Region
(B) LOCATION: 456..458
(D) OTHER INFORMATION: /note= "N-linked glycosylation
site"

(ix) FEATURE:
(A) NAME/KEY: Region
(B) LOCATION: 24..540
(D) OTHER INFORMATION: /note= "Extracellular region"

(xi) SEQUENCE DESCRIPTION: SEQ ID NO:2:

(2) INFORMATION FOR SEQ ID NO:3:

    (i) SEQUENCE CHARACTERISTICS:
        (A) LENGTH: 660 amino acids
        (B) TYPE: amino acid
        (D) TOPOLOGY: linear

   (ii) MOLECULE TYPE: protein

   (xi) SEQUENCE DESCRIPTION: SEQ ID NO:3:

Met Glu Pro Leu Val Thr Trp Val Val Pro Leu Leu Phe Leu Phe Leu
1               5                   10                  15

Leu Ser Arg Gln Gly Ala Ala Cys Arg Thr Ser Glu Cys Cys Phe Gln
            20                  25                  30

Asp Pro Pro Tyr Pro Asp Ala Asp Ser Gly Ser Ala Ser Gly Pro Arg
        35                  40                  45

Asp Leu Arg Cys Tyr Arg Ile Ser Ser Asp Arg Tyr Glu Cys Ser Trp
    50                  55                  60

Gln Tyr Glu Gly Pro Thr Ala Gly Val Ser His Phe Leu Arg Cys Cys
65                  70                  75                  80

Leu Ser Ser Gly Arg Cys Cys Tyr Phe Ala Ala Gly Ser Ala Thr Arg
                85                  90                  95

Leu Gln Phe Ser Asp Gln Ala Gly Val Ser Val Leu Tyr Thr Val Thr
            100                 105                 110

Leu Trp Val Glu Ser Trp Ala Arg Asn Gln Thr Glu Lys Ser Pro Glu
        115                 120                 125

Val Thr Leu Gln Leu Tyr Asn Ser Val Lys Tyr Glu Pro Pro Leu Gly
    130                 135                 140

Asp Ile Lys Val Ser Lys Leu Ala Gly Gln Leu Arg Met Glu Trp Glu
145                 150                 155                 160

Thr Pro Asp Asn Gln Val Gly Ala Glu Val Gln Phe Arg His Arg Thr
                165                 170                 175

Pro Ser Ser Pro Trp Lys Leu Gly Asp Cys Gly Pro Gln Asp Asp Asp
            180                 185                 190

Thr Glu Ser Cys Leu Cys Pro Leu Glu Met Asn Val Ala Gln Glu Phe
        195                 200                 205

Gln Leu Arg Arg Arg Gln Leu Gly Ser Gln Gly Ser Ser Trp Ser Lys
    210                 215                 220

Trp Ser Ser Pro Val Cys Val Pro Pro Glu Asn Pro Pro Gln Pro Gln
225                 230                 235                 240

Val Arg Phe Ser Val Glu Gln Leu Gly Gln Asp Gly Arg Arg Arg Leu
                245                 250                 255

Thr Leu Lys Glu Gln Pro Thr Gln Leu Glu Leu Pro Glu Gly Cys Gln
            260                 265                 270

Gly Leu Ala Pro Gly Thr Glu Val Thr Tyr Arg Leu Gln Leu His Met
        275                 280                 285

Leu Ser Cys Pro Cys Lys Ala Lys Ala Thr Arg Thr Leu His Leu Gly
    290                 295                 300

Lys Met Pro Tyr Leu Ser Gly Ala Ala Tyr Asn Val Ala Val Ile Ser
305                 310                 315                 320

Ser Asn Gln Phe Gly Pro Gly Leu Asn Gln Thr Trp His Ile Pro Ala
                325                 330                 335

Asp Thr His Thr Glu Pro Val Ala Leu Asn Ile Ser Val Gly Thr Asn
            340                 345                 350

Gly Thr Thr Met Tyr Trp Pro Ala Arg Ala Gln Ser Met Thr Tyr Cys
        355                 360                 365

Ile Glu Trp Gln Pro Val Gly Gln Asp Gly Gly Leu Ala Thr Cys Ser
    370                 375                 380

Leu Thr Ala Pro Gln Asp Pro Asp Pro Ala Gly Met Ala Thr Tyr Ser
385                 390                 395                 400

Trp Ser Arg Glu Ser Gly Ala Met Gly Gln Glu Lys Cys Tyr Tyr Ile
                405                 410                 415

Thr Ile Phe Ala Ser Ala His Pro Glu Lys Leu Thr Leu Trp Ser Thr
            420                 425                 430

Val Leu Ser Thr Tyr His Phe Gly Gly Asn Ala Ser Ala Ala Gly Thr
        435                 440                 445

Pro His His Val Ser Val Lys Asn His Ser Leu Asp Ser Val Ser Val
    450                 455                 460

Asp Trp Ala Pro Ser Leu Leu Ser Thr Cys Pro Gly Val Leu Lys Glu
465                 470                 475                 480

Tyr Val Val Arg Cys Arg Asp Glu Asp Ser Lys Gln Val Ser Glu His
                485                 490                 495

Pro Val Gln Pro Thr Glu Thr Gln Val Thr Leu Ser Gly Leu Arg Ala
            500                 505                 510

Gly Val Ala Tyr Thr Val Gln Val Arg Ala Asp Thr Ala Trp Leu Arg
        515                 520                 525

Gly Val Trp Ser Gln Pro Gln Arg Phe Ser Ile Glu Val Gln Val Ser
    530                 535                 540

Asp Trp Leu Ile Phe Phe Ala Ser Leu Gly Ser Phe Leu Ser Ile Leu
545                 550                 555                 560

Leu Val Gly Val Leu Gly Tyr Leu Gly Leu Asn Arg Ala Ala Arg His
                565                 570                 575

Leu Cys Pro Pro Leu Pro Thr Pro Cys Ala Ser Ser Ala Ile Glu Phe
            580                 585                 590

Pro Gly Gly Lys Glu Thr Trp Gln Trp Ile Asn Pro Val Asp Phe Gln
        595                 600                 605

Glu Glu Ala Ser Leu Gln Glu Ala Leu Val Val Glu Met Ser Trp Asp
    610                 615                 620

Lys Gly Glu Arg Thr Glu Pro Leu Glu Lys Thr Glu Leu Pro Glu Gly
625                 630                 635                 640

Ala Pro Glu Leu Ala Leu Asp Thr Glu Leu Ser Leu Glu Asp Gly Asp
                645                 650                 655

Arg Cys Asp Arg
            660